# EUROPEAN PATENT APPLICATION

(11) **EP 1 072 609 A2**
(43) Date of publication of application: **31.01.2001**
(21) Application number: 00305504.3
(22) Date of filing: 30.06.2000
(51) Int. Cl.: C07K 14/475, A61K 38/18

(54) **Cytoprotective agents comprising prosaposin-related peptides**

(30) Priority: 30.06.1999 JP 18515599
(71) Applicant: Sakanaka, Masahiro, Onsen-gun, Ehime-ken 971-0204 (JP); Tanaka, Junya, Onsen-gun, Ehime-ken 791-0203 (JP); Sato, Kohji, Hamamatsu-shi, Shizuoka-ken 430-0805 (JP)
(72) Inventor: Sakanaka, Masahiro, Onsen-gun, Ehime-ken 791-0204 (JP); Tanaka, Junya, Onsen-gun, Ehime-ken 791-0203 (JP); Sato, Kohji, Hamamatsu-shi, Shizuoka-ken 430-0805 (JP); Morita, Fumio, Tokushima-shi, Tokushima-ken 770-0044 (JP); Sadamoto, Yatsutaka, Niihama-shi, Ehime-ken 792-0005 (JP)
(74) Representative: Cresswell, Thomas Anthony

(57) **Abstract**

Prosaposin-related peptides or derivatives thereof are useful as cytoprotective agents, for suppression of apoptosis or apoptosis-like cell death and for promoting the expression of cell death-suppressing or anti-apoptotic gene product Bcl-X_{L}.

### Annex to the application documents - subsequently filed sequences listing

SEQUENCE LISTING

<110> SAKANAKA, Masahiro
      TANAKA, Junya
      SATO, Kohji

<120> CYTOPROTECTIVE AGENTS COMPRISING PROSAPOSIN-RELATED
      PEPTIDES

<130> N79825 TAC AB

<140> 00305504.3
<141> 2000-06-30

<150> JP 11-185155/1999
<151> 1999-06-30

<160> 20

<170> PatentIn Ver. 2.1

<210> 1
<211> 8
<212> PRT
<213> Artificial Sequence

<220>
<221> VARIANT
<222> (1)
<223> Xaa is Leu or Ile

<220>
<221> VARIANT
<222> (2)
<223> Xaa is  1, 2 or 3 amino acid residues, each being
      any possible amino acid residue

<220>
<221> VARIANT
<222> (5)
<223> Xaa is 1 to 7 amino acid residues, each being any
      possible amino acid residue

<220>
<221> VARIANT

<222> (6)
<223> Xaa is 1, 2 or more residues which are Asp, Lys,
      Glu or Arg

<220>
<221> VARIANT
<222> (7)
<223> Xaa is 5 to 9 amino acid residues

<220>
<221> VARIANT
<222> (7)
<223> Xaa is 1, 2 or more amino acid residues which are
      Ala, Leu, Ile or Val

<220>
<223> Description of Artificial Sequence: Peptide

<400> 1
Xaa Xaa Asn Asn Xaa Xaa Xaa Xaa
  1               5

<210> 2
<211> 6
<212> PRT
<213> Artificial Sequence

<220>
<223> Description of Artificial Sequence: Peptide

<220>
<221> VARIANT
<222> (3)
<223> Xaa is any amino acid

<220>
<221> VARIANT
<222> (6)
<223> Xaa is any amino acid

<400> 2
Leu Ile Xaa Asn Asn Xaa
  1               5

<210> 3
<211> 18
<212> PRT
<213> Artificial Sequence

<220>
<223> Description of Artificial Sequence: Peptide

<400> 3
Leu Ser Glu Leu Ile Ile Asn Asn Ala Thr Glu Glu Leu Leu Ile Lys
1               5                   10                  15

Gly Leu

<210> 4
<211> 25
<212> PRT
<213> Homo sapiens

<400> 4
Cys Glu Phe Leu Val Lys Glu Val Thr Lys Leu Ile Asp Asn Asn Lys
1               5                   10                  15

Thr Glu Lys Glu Ile Leu Asp Ala Phe
            20                  25

<210> 5
<211> 25
<212> PRT
<213> Mus musculus

<400> 5
Cys Gln Phe Val Met Asn Lys Phe Ser Glu Leu Ile Val Asn Asn Ala
1               5                   10                  15

Thr Glu Glu Leu Leu Tyr Lys Gly Leu
            20                  25

<210> 6
<211> 25
<212> PRT
<213> Rattus rattus

<400> 6
Cys Gln Leu Val Asn Arg Lys Leu Ser Glu Leu Ile Ile Asn Asn Ala
1               5                   10                  15

Thr Glu Glu Leu Leu Ile Lys Gly Leu
            20                  25

<210> 7
<211> 25
<212> PRT
<213> guinea pig

<400> 7
Cys Glu Tyr Val Val Lys Lys Val Met Leu Leu Ile Asp Asn Asn Arg
1               5                   10                  15

Thr Glu Glu Lys Ile Leu His Ala Leu
            20                  25

<210> 8
<211> 25
<212> PRT
<213> pig

<400> 8
Cys Glu Phe Val Val Lys Glu Val Ala Lys Leu Ile Asp Asn Asn Arg
1               5                   10                  15

Thr Glu Glu Glu Ile Leu His Ala Leu
            20                  25

<210> 9
<211> 22
<212> PRT
<213> Homo sapiens

<400> 9
Cys Lys Asp Val Val Thr Ala Ala Gly Asp Met Leu Lys Asp Asn Ala
1               5                   10                  15

Thr Glu Glu Glu Ile Leu
            20

<210> 10
<211> 20
<212> PRT
<213> Homo sapiens

<400> 10
Tyr Val Lys His Gln Gly Leu Asn Lys Asn Ile Asn Leu Asp Ser Val
1               5                   10                  15

Asp Gly Val Pro
            20

<210> 11
<211> 15
<212> PRT
<213> Homo sapiens

<400> 11
Glu Ala Leu Ala Glu Asn Asn Leu Asn Leu Pro Lys Met Ala Gly
1               5                   10                  15

<210> 12
<211> 17
<212> PRT
<213> Homo sapiens

<400> 12
Leu Gln Met Ile Leu Asn Gly Ile Asn Asn Tyr Lys Asn Pro Lys Leu
1               5                   10                  15

Thr

<210> 13
<211> 12
<212> PRT
<213> Homo sapiens

<400> 13
Ile Leu Met Glu Asn Asn Leu Arg Arg Pro Asn Leu
1               5                   10

<210> 14
<211> 13
<212> PRT
<213> Homo sapiens

<400> 14
Phe Tyr Leu Arg Asn Asn Gln Leu Val Ala Gly Thr Leu
1               5                   10

<210> 15
<211> 16
<212> PRT
<213> Homo sapiens

<400> 15

<210> 16
<211> 19
<212> PRT
<213> Homo sapiens

<400> 16
Tyr Thr Ala Gln Gly Glu Pro Phe Pro Asn Asn Val Glu Leu Lys Leu
1               5                   10                  15

Cys Ala Pro

<210> 17
<211> 15
<212> PRT
<213> Homo sapiens

<400> 17
Phe Asn Lys Ile Glu Ile Asn Asn Lys Leu Glu Phe Glu Ser Ala
1               5                   10                  15

<210> 18
<211> 18
<212> PRT
<213> Homo sapiens

<400> 18

<210> 19
<211> 22
<212> PRT
<213> Artificial Sequence

<220>
<223> Description of Artificial Sequence: Consensus
      sequence

<400> 19
Cys Xaa Xaa Xaa Val Xaa Xaa Xaa Xaa Xaa Leu Ile Xaa Asn Asn Xaa
1               5                   10                  15

Thr Glu Xaa Xaa Ile Xaa
            20

<210> 20
<211> 22

<212> PRT
<213> Homo sapiens

<400> 20
Cys Glu Phe Leu Val Lys Glu Val Thr Lys Leu Ile Asp Asn Asn Lys
1               5                   10                  15

Thr Glu Lys Glu Ile Leu
            20

## Description

### TECHNICAL FIELD OF THE INVENTION

The present invention relates to prosaposin-related peptides or derivatives thereof. More particularly, the present invention pertains to a pharmaceutical composition comprising prosaposin-related peptides or derivatives thereof which suppress apoptosis or apoptosis-like cell death, or the pharmaceutical composition comprising-prosaposin-related peptides or derivatives thereof that promote the expression of cell death-suppressing or antiapoptotic gene product Bcl-x_{L}.

### BACKGROUND OF THE INVENTION

Prosaposin is the sphingolipid hydrolase activator protein precursor and the sphingolipid hydrolase activator protein is composed of four domains (saposins A, B, C and D)(O'Brien, J.S. et al., Science, 241, 1098-1101, 1988; Kishimoto, Y. et al., J. Lipid Res., 33, 1255-1267, 1992; and O'Brien, J.S. and Kishimoto, Y. FASEB J., 5, 301-308, 1991). In peripheral tissues such as the liver, spleen and kidneys, saposins A, B, C and D are processed from prosaposin to activate enzymes involved in the metabolism of sphingolipid (for example, β-glucosylceramidase, β-galactosylceramidase, arylsulphatase A, globotriaosylceramide α-galactosidase, GM1-β-galactosidase and sphingomyelinase). Deficiency of these saposins is known to cause a disease gangliosidosis.

In the brain, prosaposin is not so frequently subjected to proteolysis, unlike the case in the above mentioned peripheral tissues, and it is located in specific central regions including the hippocampus in the form of the precursor (Kondoh, K et al., J. Comp. Neurol., 334, 590-602, 1993; and Sano, A. et al., Biochem. Biophys. Res. Commun., 165, 1191-1197, 1989).

Recently, it becomes clear that prosaposin itself has a neurotrophic action without affecting sphingolipid hyrolase activity, in addition to acting as the precursor of saposins A, B, C and D (O'Brien, J.S. et al., Proc. Natl. Acad. Sci. USA, 91, 9593-9596, 1994; Sano, A. et al., Biochem. Biophys. Res. Commun., 204, 994-1000, 1994). Furthermore, it is proven that an active center responsible for the neurotrophic action of prosaposin is a peptide consisting of 18 amino acid residues (18-mer peptide, molecular weight approximately 2000) in the hydrophilic region of human and rat saposin C domains (U.S. Patent Nos. 5,571,787, 5,696,080, 5,700,909, and 5,714,459; Kotani, Y. et al., J. Neurochem. 66, 2197-2200, 1996).

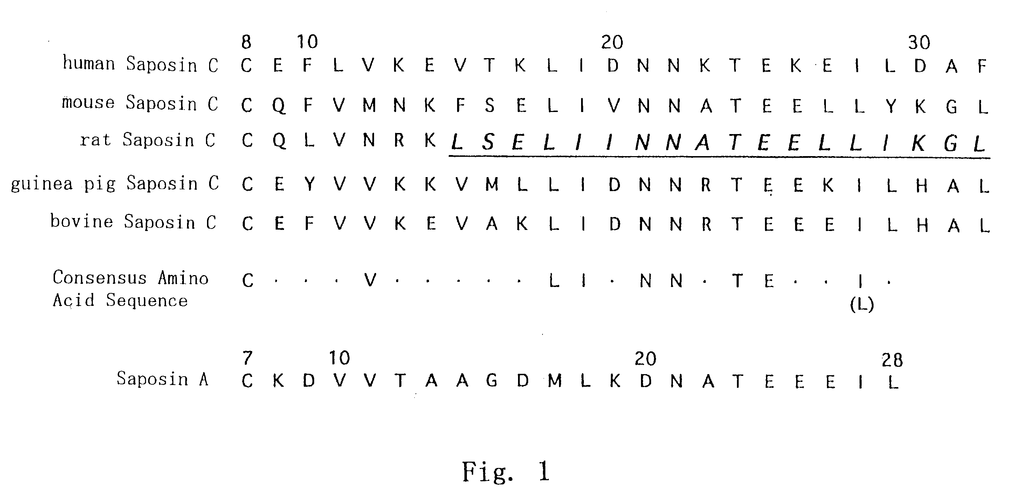

Fig. 1 shows amino acid sequences consisting of 25 amino acid residues containing 18-mer peptide of human, rat, guinea pig and bovine saposin C domains. In Fig. 1, the underlined part is the rat 18-mer peptide. Consensus amino acid sequence shows an amino acid sequence common to the amino acid sequences of these saposin C domains.

Hereinafter, as shown in Fig. 1, in the present specification, prosaposin-related 18-mer peptides or their derivatives of various animal origin having the common aminoacid sequence, i.e. the consensus sequence (LIXNNX, wherein X is any one of aminoacids, and each alphabetical symbol shows single-letter symbol of amino acid), and peptides or their derivatives consisting of amino acid residues more or less than 18-mer peptide having the said consensus amino acid sequence, are totally designated as 18-MP.

These peptides having the consensus amino acid sequence are known substances described in the U.S. Patents hereinbefore mentioned. The 18-MP having the neurotrophic activity does not almost involved in the above mentioned sphingolipid hydrolase activation. Consequently, heretofore, almost no attention has been paid to the physiological action of a precursor of the functional protein itself, however as a result of the above studies, it has been demonstrated that prosaposin (precursor of saposins) or 18-MP had neurotrophic activity which was completely different nature from saposins.

The fact that 18-MP, like prosaposin, activates intracellular signal transduction through binding to the receptor on the surface of cells is disclosed in the U.S. Patents and others hereinbefore mentioned. In the U.S. Patents hereinbefore, it is disclosed that 18-MP facilitates process elongation in NS20Y neuroblastoma at the concentration range between 0.01 - 0.5 µg/ml.

On the other hand, we have found that 18-MP protected the primary cultured neurons exposed to hypoxic condition at the same concentration range or a slightly lower concentration range (200 pg/ml - 20 ng/ml)(Igase, K. et al., J. Cereb. Blood Flow Metab., 19, 298-306, 1999). Further, we have found that necrosis of neurons by an action of a free radical inducer (ferrous sulfate) can be significantly reduced by preadministration of 18-MP at a low concentration-range (2 fg/ml - 2 pg/ml)unexpected until now (Igase, K. et al., J. Cereb. Blood Flow Metab., 19, 298-306, 1999).

These facts indicate that, in the concentration range (0.01 - 0.5 µg/ml) described in the above U.S. Patents or in the concentration range which can improve hypoxic damage (200 pg/ml - 20 ng/ml), 18-MP exhibits its physiological action by mainly binding with low affinity receptor, and in the far lower concentration range (2 fg/ml - 2 pg/ml) than that, 18-MP may prevent necrosis of cells caused by the free radical inducer ferrous sulfate, by binding with high affinity receptor.

However, it still remains in question whether experimental results using the free radical inducer (ferrous sulfate) reflected directly the action mechanism of 18-MP in in vivo brain tissue or not. We (Sakanaka and Tanaka) have found that 18-MP significantly suppressed apoptosis-like neuron death in the ischemic penumbra of the cerebral cortex only by continuous infusion to the left lateral ventricle after cerebral ischemia in a dose of 20 - 60 ng/day for 28 days in the permanent occlusion model of the middle cerebral artery distal to the striate branches (MCA) of stroke-prone spontaneously hypertensive rats (SH-SP rats)(so called cerebral infarction-rat)(Igase, K. et al., J. Cereb. Blood Flow Metab., 19, 298-306, 1999). Judging from the experimental results of the above mentioned report, most of the nerve cells or neurons in the ischemic penumbra might be rescued from apoptosis-like death by intracerebroventricular administration of 18-MP (20 - 60 ng/day) after ischemia. In this connection, since without any treatment the neurons in the ischemic penumbra may rapidly enter apoptosis-like death within about one hour after permanent occlusion of the MCA, an extremely low concentration of 18-MP infused intraventricularly (it may be around 2 fg/ml - 2 pg/ml at a level of extracellular fluid) appears to accumulate close to damaged neurons in the ischemic penumbra to rescue them from death. In this occasion, it is in question whether or not the low extracellular level of 18-MP protects the neurons, as suggested in the above cultured experiments, by merely reducing the free radical injury. Generally, it is known that, in the re-perfusion of cerebral ischemia, large amounts of oxygen free radicals are generated to damage neurons in situ. However, since it is difficult to say that large amounts of oxygen free radicals are produced in the ischemic penumbra of animals with permanent occlusion of the MCA, the possibility that certain neurotoxic or proapoptotic factors other than oxygen free radicals are involved in the apoptosis-like neuron death in the ischemic penumbra is high. Consequently, in the ischemic penumbra of animals with permanent MCA occlusion, low extracellular levels of 18-MP (presumably around 2 fg/ml - 2 pg/ml of 18-MP) may suppress apoptosis-like neuron death through a mechanism(s) other than reducing the toxicity of oxygen free radicals. As described in the Japanese Patent Application No. Hei 10-365560 (Protective agents for brain cells or nerve cells comprising ginsenoside Rb₁), in this specification, slowly progressing neuron death, which is different from necrosis, is defined as apoptosis of neurons or apoptosis-like neuron death.

As explained hereinabove, an unsolved problem still remains on the action mechanism of 18-MP. We have found that 18-MP increases the expression of cell death-suppressing or antiapoptotic gene product Bcl-x_{L} in neurons or cardiac muscle cells and suppresses apoptosis of neurons or cardiac muscle cells or apoptosis-like death of neurons or cardiac muscle cells at the far lower extracellular level [i.e. 1 ng/ml (approximately 0.5 nM) or less, preferably 10 pg/ml (approximately 5 pM) or less, and more preferably 100 fg/ml (approximately 5 fM) or less] than the level at approximately 0.01 - 0.5 µg/ml, which is disclosed in the previous U.S. Patents (U.S. Patent No. 5,571,787, Prosaposin as a neurotrophic factor; U.S. Patent No. 5,696,080, Pharmaceutical compositions comprising neurotrophic peptide derived from prosaposin; U.S. Patent No. 5,700,909, Prosaposin and cytokine-derived peptides; U.S. Patent No. 5,714,459, Use of prosaposin and neurotrophic peptides derived therefrom), and completed the present invention.

An object of the present invention is to provide pharmaceuticals that protect cells by promoting the expression of cell death-suppressing or antiapoptotic gene Bcl-x_{L}.

Further object of the present invention is to provide an effective preparation for administration of 18-MP or salt thereof which is useful as cytoprotective agents. More-further object of the present invention is to provide a pharmaceutical composition for suppression of apoptosis or apoptosis-like cell death comprising 18-MP or salt thereof, or a pharmaceutical composition for promoting the expression of cell death-suppressing or antiapoptotic gene product Bcl-x_{L} comprising 18-MP or salt thereof.

### DESCRIPTION OF THE INVENTION

The present invention relates to a pharmaceutical composition for suppressing apoptosis or apoptosis-like cell death comprising low concentrations of 18-MP or salt thereof and pharmaceutical acceptable carrier.

The present invention relates to a pharmaceutical composition comprising 18-MP or salt thereof and pharmaceutical acceptable carrier for promoting the expression of cell death-suppressing or antiapoptotic gene product Bcl-x_{L}.

The present invention relates to a method for treatment of the diseases involving apoptosis or apoptosis-like cell death comprising to administer the effective amount of low concentrations of 18-MP or salt thereof to a patient.

The present invention relates to use of 18-MP or salt thereof for a manufacture-a pharmaceutical composition for suppressing apoptosis or apoptosis-like cell death or promoting the expression of cell death-suppressing or antiapoptotic gene product Bcl-xL.

18-MP of the present invention is prosaposin-related peptides having the above described consensus amino acid sequence (LIXNNX, wherein X is any one of amino acids) (Fig. 1), that is, one or two kinds of prosaposin-related peptide or derivative thereof at least consisting of partial amino acid sequence of the formula " Leu-Ile-Xaa-Asn-Asn-Xaa" wherein Xaa is any one of aminoacid and pharmaceutical acceptable carrier and 18-MP can be synthesized by using an automatic peptide synthesizer.

18-MP of the present invention can be used in its free form as well as preferable salt thereof. It can also be used as their solvates such as hydrates.

The concentration of 18-MP used in the present invention is preferably low, more concretely the extracellular levels in lesion tissue are 1 ng/ml (0.5 nM) or less, preferably 10 pg/ml (0.5 pM) or less, and more preferably 100 fg/ml (50 fM) or less. In case that 18-MP of the present invention is used in the form of preparation for intravenous administration, preparation for intraventricular administration, external preparation for local lesion, injection for local lesion, preparation for oral administration, nasal drop preparation, suppositories, injection for subcutaneous administration, injection for intracutaneous administration, preparation for intramuscular administration, preparation for inhalation, preparation for sublingual administration and preparation for preparation for percutaneous absorption, these preparations are preferably prepared so that the extracellular levels of 18-MP in the lesion tissue of patients are maintained at the above mentioned concentrations. Further, 18-MP can be coated with microgranules of polymers and are selected for any administration routes. Sufficient cytoprotective effects or promotion of Bcl-x_{L} expression of the pharmaceutical composition and preparation of the present invention can be obtained even at the extracellular concentrations of around 1 - 100 fg/ml in the lesion. It is one of specific features that the use of low concentrations of 18-MP is preferable.

18-MP of the present invention can suppress apoptosis-like neuron death, if at least a daily dose of 20 ng is administered intracerebroventricularly in Mongolian gerbil (Meriones umguiculatus, body weight 70 - 80 g). Since approximately 0.03% of intravenously administered 18-MP may be transferred into the brain (Banks, W. et al., Peptides 13, 1289-1294, 1992), if approximately 66.7 µg/day or more of 18-MP are intravenously administered in Mongolian gerbil, apoptosis-like neuron death may be prevented. Since the average body weight of Mongolian gerbil is 75 g, dose amount of 18-MP per kg of body weight in a day is approximately 888.9 µg or more/kg/day. In the example 1 of the present invention, although 18-MP, 20 ng/day/head, is administered intracerebroventricularly in Mongolian gerbil, numbers of TUNEL-positive cells in the hippocampal CAI region, i.e. frequency of apoptosis-like neuron death, can be reduced only by approximately 30%, then actual administration of 18-MP with a dosage of 20 ng/day or more is expected to exhibit a more preferable suppressive effect on apoptosis-like neuron death. Consequently, in case that therapy, prevention or treatment of diseases accompanying with apoptosis-like neuron death are performed by intravenous administration of 18-MP, a daily dose is preferably 1000 µg (1 mg) or more/kg of body weight. Amount of intravenous administration of 18-MP at 1 mg or more and 100 mg or less per kg of bodyweight may be able to applicable for therapy, prevention or treatment of brain diseases accompanied with apoptosis-like neuron death (cerebral apoplexy, stroke, neurodegenerative diseases, brain injury, spinal cord injury and cerebral-palsy). The said amount of administration is exceeded the estimated dose amount in the U.S. Patent (No. 5,571,787, Prosaposin as a neurotrophic factor)(0.1 -1000 µg/kg).

As explained hereinbefore, far lower levels of 18-MP in the extracellular fluid can prevent apoptosis-like neuron death. However, since intracerebral transfer rate of 18-MP administered intravenously is extremely low, in order to apply 18-MP to therapy, prevention or treatment of brain diseases, intravenous dose amounts more than the expected maximal dose (1000 µg/kg) disclosed in the U.S. Patent may be required. On the other hand, the present invention discloses as a pioneer in the world that 18-MP suppresses apoptosis or apoptosis-like cell death at the unprecedentedly low levels of concentration among the prior known peptide factors by promoting the expresion of Bcl-x_{L} protein. Since Bcl-x_{L} is prevalently expressed not only in brain cells (neurons, glia) but also in cells of almost all peripheral tissues such as the skin, liver, hypophysis, kidney, heart, salivary gland, digestive tube, blood vessel, connective tissue, blood, bone marrow, lung, pancreas, trachea, brouchus, tongue, oral cavity, thyroid gland, adrenal gland, uterus, gallbladder, urinary bladder, parathyroid gland, ureter, ovary, testis, ear, eyeball, nose, bone, vagina, cartilage, tendon, joint, pharynx, larynx, tooth and muscle, the fact that 18-MP suppresses apoptosis or apoptosis-like cell death by promoting the expression of Bcl-x_{L} protein indicates effectiveness of 18-MP for not only central nervous system diseases-but also for peripheral tissue diseases accompanied by apoptosis-like cell death or apoptosis (for example, rejection after organ transplantation, contact dermatitis, hand and finger dermatitis, senile xerosis, asteatotic dermatitis, nummular eczema, autosensitization dermatitis, seborrheic dermatitis, dundruff, erythroderma, generalized exfoliative dermatitis, urticaria, cutaneous pruritus, insect bite, strophulus, prurigo, purpura, progressive pigmented purpuric dermatitis, Shamberg disease, erythema exsudativum multiforme, annular erythema, erythema nodosum, pernio, chilblain, drug eruption, photosensitivity, epidermolysis bullosa, pemphigus, bullous pemphigoid, dermatitis herpetiformis, pustulosis palmaris et plantaris, psoriasis, lichen planus, ichthyosis, lichen pilaris xanthomatosis, cutaneous amyloidosis, herpes simplex, herpes zoster, viral wart, molluscum contagiosum, pyoderma, skin-tuberculosis, atypical mycobacteriosis of the skin, tinea, cutaneous candidiasis, oral candidiasis, scabies, pediculosis pubis, syphilis, keloid, hypertrophic scar, angioma, lymphangioma, vitiligo vulgaris, ephelides, chloasma, melanosis, Pompholyx, miliaria, osmidrosis, acne, rosacea, rosacea-like dermatitis, perioral dermatitis, alopecia, leg ulcer, ischemia re-perfusion injury of cardiac muscle, liver and kidneys, alcoholic liver disease, hepatitis, nephritis, fatty liver, collagen diseases, drug-induced liver injury, myocardial infarction, silent myocardial ischemia, cardiac insufficiency, heart failure, cardiomyopathy, occlusion of peripheral arteries, burn, cold injury, arrythmia, thromboangitis obliterans, radiation injury, ultraviolet ray injury, arteriosclerosis, arteriosclerosis obliterans, traumatic wound, non-traumatic wound, postoperational wound, angina pectoris, peripheral circulation insufficiency, shock, bedsore, corneal injury, skin injury, wound, atopic dermatitis, autoimmune diseases, diabetic angiopathy, diabetes mellitus, diabetic nephropathy, renal failure, hepatic failure, AIDS, immunodeficiency disease, skin ulcer, and peptic ulcer). Thus, it is likely that 18-MP is useful-for the therapy, prevention or treatment of any diseases involving cell death, except psychiatric disorders such as schizophrenia and depression. The other diseases involving cell death not mentioned in this specification are described in a book entitled Today's Therapy 1997 (edited by Inagaki, Y., Takasu, Y., Ogata, E., under the supervision of Hinohara, S. and Abe, M., Igaku-shoin Ltd. Tokyo, 1997). Further, low concentrations of 18-MP appear to be effective for protection and maintenance of keratinocytes cultured for skin grafting. In the other organs for transplantation (liver, kidney, heart, pancreas, lung, digestive tract, cornea, blood vessel, and others), the organs can be dipped in or perfused with the solution of low concentrations of 18-MP during, before or after the transplantation, and thereby cell death of the grafted tissues is suppressed and the organs are protected. Quite naturally, when 18-MP is used for therapy, prevention and treatment of diseases of the peripheral organs and tissues, the amount of administration can be smaller than the administration for therapy, prevention and treatment of brain diseases, and in that case the extracellular level of 18-MP in the lesioned tissue is required to be kept considerably lower than the level (0.01 - 0.5 µg/ml) disclosed in the U.S. Patent (No. 5,571,787, Prosaposin as a neurotrophic factor). If we assume that the molecular weight of 18-MP is approximately 2000, appropriate daily systemic dosages of 18-MP for the therapy, prevention or treatment of peripheral organ (and tissue) diseases are in the range of from about 0.001 to about 100 µg/kg, based on the body weight of the vertebrate, although dosages from about 0.00001 to about 1000 µg/kg are also contemplated. Daily dosages of locally administered material (18-MP) will be in the range of from about 0.00001 fg/kg to about 20 µg/kg, preferably in the range of from about 0.00001 fg/kg about 0.1 µg/kg, and more preferably in the range of from about 0.00001 fg/kg, depending on the size and/or severity of local lesion either in peripheral tissues and organs or in the central nervous system. Local administration of the present pharmaceutical composition before, during and/or after surgical operation in humans, domestic animals and pets is highly recommended. For example, if we locally administer 100 ml of artificial cerebrospinal fluid containing 18-MP at the concentration of 100 fg/ml during a neuronsurgical operation in a patient weighing 50kg, the total amount of 18-MP needed will be approximately 10 pg. Therefore, in the patient the daily dosage of locally administered 18-MP will be around 0.2 pg/kg, based on the body weight of the patient. If we apply 0.1g of ointment containing 18-MP at the concentration of 1 fg/ml(g) to a tiny traumatic wound in the skin of another patient weighting 50kg, the daily dosage of locally administered 18-MP will be 0.002 fg/kg. However, the above dosages of systemically or locally administered 18-MP should be adjusted so that the extracellular concentrations of the pharmaceutical composition in lesion tissue are kept at 1 ng/ml or less, preferably at 10 pg/ml or less, and more preferably at 100 fg/ml or less. Oral administration may be possible if 18-MP is stable to gastrointestinal degradation and readily absorbed.

Any methods for administration of the pharmaceutical composition of the present invention can be selected, as long as the extracellular levels of 18-MP in the lesioned tissues can be kept at 1 ng/ml (0.5 nM) or less, preferably 10 pg/ml (5 pM) or less, more preferably 100 fg/ml (50 fM) or less. To be more specific, intravenous administration, intraventricular administration, subcutaneous administration, local infusion in lesioned tissues, intramuscular injection, external use in lesioned tissues, eye drops, eye ointment, nasal drops, ear drops, ear ointment, inhalation, suppositories, oral administration, sublingual administration and percutaneous administration can be selected. 18-MP of the present invention can be formulated by the conventional methods. For example, the water-soluble pharmaceutical composition of the present invention can be prepared for administration form by dissolving the lyophilized crystals in physiological saline, distilled water, phosphate buffer or glucose solution. External use for skin and suppositories can be prepared by mixing 18-MP with water-soluble or fat-soluble base. Lipid suspension and liposome preparation can also be used. Administration can also be performed by combination with carrier without biological adverse effects. Concentrations of 18-MP in the formulation for administration can be adjusted in any ways unless they are high, for example 0.01 - 10 mg/ml, preferably 0.1 - 1 mg/ml. Also, 18-MP is administered locally to lesioned peripheral tissues in vivo by implantation of the material. For example, polyactic acid, polygalactic acid, regenerated collagen, multilamellar liposomes and many other conventional depot formulations comprise bioerodible or biodegradable materials that can be formulated with biologically active compositions. These materials, when implanted, gradually break down and release the active material (18-MP) to the surrounding tissue. The use of bioerodible, biodegradable and other depot formulations is expressly contemplated in the present invention. Infusion pumps, matrix entrapment systems, and combination with transdermal delivery devices are also contemplated.

As described above, 18-MP of the present invention may also advantageously be enclosed in micelles or liposomes. Liposome encapsulation technology is well known. Liposomes may be targeted to specific peripheral tissues or organs through the use of receptors, ligands or antibodies capable of binding the targeted tissues or organs.

The composition of the present invention can be used in vitro and in vivo as research tools for studying the cytoprotective effects of anti-apoptotic agents. However, more practically, 18-MP has an immediate use as laboratory reagents and components of cytoprotection media in order to better enable survival of any types of cells in vitro. Finally, 18-MP with a potent cytoprotective action can be used as an ingredient or a composition of any cosmetics to prevent or improve the symptoms of ageing of the skin, such as atrophy, wrinkling, chap, sagging, laxity, vulnerability to infection, exfoliation, dryness, itching, gray hair, alopecia and mottled pigmentation. The concentration of 18-MP in a cosmetic is 0.1% or less, preferably 0.001% or less, more preferably between 10⁻⁵ % and 10⁻¹⁵ % by weight, based on the total weight of the composition. The uppermost concentration of 18-MP in any cosmetics is 1%. However, the above concentrations of 18-MP in cosmetics should be adjusted so that the extracellular concentrations of the cosmetic composition in the skin are kept at 1 ng/ml or less, preferably at 10 pg/ml or less, and more preferably at 100 fg/ml or less.

### BRIEF DESCRIPTION OF THE FIGURES

Fig. 1 shows partial amino acid sequences of saposin C domain and partial amino acid sequence of saposin A of various animals. In the figure, numerals indicate amino acid number calculated from N terminal. According to the U.S. Patens (U.S. Patent No. 5,571,787, Prosaposin as a neurotrophic factor; U.S. Patent 5,696,080, Pharmaceutical compositions comprising neurotrophic peptide derived from prosaposin; U.S. Patent No. 5,700,909, Prosaposin and cytokine-derived peptides; U.S. Patent No. 5,714,459, Use of prosaposin and neurotrophic peptides derived therefrom), only saposin C but not saposin A stimulates, neurite outgrowth. Consequently, consensus amino acid sequence specific to saposin C (LIXNNX, wherein X is any one of amino acids) appears to be responsible for common physiological actions of 18-MP. 18-MP used in examples of the present invention is indicated by underlined boldface.

Fig.2 shows TUNEL-positive cells and their numbers in the hippocampal CA1 field. "A" shows the vehicle administration after 3-minutes ischemia. "B" shows 18-MP administration after 3-minutes ischemia (20 ng/day). "C" shows the graph showing significant reduction of TUNEL-positive cells by administration of 18-MP. Bar indicates 100 µm.

Fig. 3 shows photomicrographs showing hippocampal CA1 regions of animals with sham operation and on 7 days, one month, 3 months, 6 months and one year after 3-minutes ischemia.

Fig. 4 shows neuronal density in the hippocampal CA1 pyramidal cell layer (A); response latency in passive avoidance learning experiment (B); and photomicrographs of the hippocampal CA1 field (C, D, E and F) are shown. When 18-MP (20 ng/day) is administered for 4 days or 28 days from postischemic day 3, degeneration or loss of hippocampal CA1 pyramidal neurons is reduced as compared with the vehicle administered group, and response latency in passive avoidance learning experiment is prolonged. Bar indicates 100 µm.

Fig. 5 shows the graph showing that 18-MP suppresses apoptosis-like neuron death caused by SNP. Left side in Fig. 5 shows experimental results without SNP treatment, and right side shows results of SNP treatment. Closed or black column: 18-MP added before and after SNP treatment. Slanting column: 18-MP added after SNP treatment.

Fig. 6 shows the result (photograph) of western blotting showing the effect of 18-MP on the expression of Bcl-x_{L} protein in neurons.

Fig. 7 shows the graph of quantitative analysis of western blotting showing the effect of 18-MP on the expression of Bcl-x_{L} protein in neurons.

Fig. 8 shows the photograph showing a stimulating effect of erythropoietin (EPO) on the expression of Bcl-x_{L} mRNA in neurons.

Fig. 9 shows the photograph showing a stimulating effect of erythropoietin (EPO) on the expression of Bcl-x_{L} protein in neurons.

Fig. 10 shows the photograph showing a stimulatiny effect of 18-MP on the expression of Bd-x_{L} protein in cardiac muscle cells (cardiac myocytes). Expression of troponin T is used as an internal standard.

Fig. 11 shows the quantitative analysis of western blotting showing the effect of 18-MP on the expression of Bcl-x_{L} protein in cardiac muscle cells (cardiac myocytes).

Fig. 12 shows the photograph of western blotting showing the expression of a striated muscle-specific protein α-actinin as a result of long-term survival by 18-MP of cardiac muscle cells (cardiac myocytes) cultured in glucose-free medium.

### DETAILED DESCRIPTION OF THE INVENTION

Actions of 18-MP of the present invention are explained in detail as follows. We have investigated whether 18-MP can suppress apoptosis-like neuron death in the hippocampus CA1 region of Mongolian gerbil which was loaded with transient forebrain ischemia of 3-minutes duration (hereinafter sometimes designates as 3-minutes transient forebrain ischemia, and the like). It is reported that Mongolian gerbils show a loss of nearly one half of pyramidal neurons in the hippocampus CA1 region at 7 days after 3-minutes transient forebrain ischemia (Sakanaka, M. et al., Proc. Natl. Acad. Sci. USA, 95, 4635-4640, 1998). In the surviving hippocampal CA1 neurons at this moment, fragmentation of nuclei, which is an index of apoptosis-like neuron death, is further in progress. This fact was confirmed by using TUNEL (terminal deoxynucleotidyltransferase-mediated 2'-deoxyuridine 5'-triphosphate-biotin nick end labeling) staining by the inventors (Sakanaka and Tanaka)(Wen, T.-C. et al., J. Exp. Med., 188, 635-649, 1998; Peng, H. et al., J. Cereb. Blood Flow Metab., 18, 349-360, 1998). With the use of the same animal model, we investigated whether apoptosis-like neuron death occurring at 7 days after 3 minutes transient forebrain ischemia can be suppressed by intracerebroventricular administration of 18-MP. 18-MP used in the following experiments is derived from rats (LSELIINNATEELLIKGL)(Kotani, Y. et al., J. Neurochem. 66, 2197-2200, 1996).

Mongolian gerbils were loaded with 3-minutes transient forebrain ischemia under inhalation anesthesia. Three days (72 hours) later, 18-MP (20 ng/day) was continuously infused into the left lateral ventricle through an osmotic minipump for 4 days. One week after 3-minutes transient forebrain ischemia, they were perfused transcardially with 0.1M phosphate buffer containing 4% paraformaldehyde under pentobarbital anesthesia, and then the brain was dissected out. The brain was embedded in paraffin, sections 5 µm thick were cut and they were subjected to TUNEL staining as described elsewhere (Wen, T.-C. et al., J. Exp. Med., 188, 635-649, 1998). In control animals with 3-minutes transient forebrain ischemia, an equal amount of vehicle (0.01 M phosphate buffered-saline containing 0.1% bovine serum albumin) was infused.

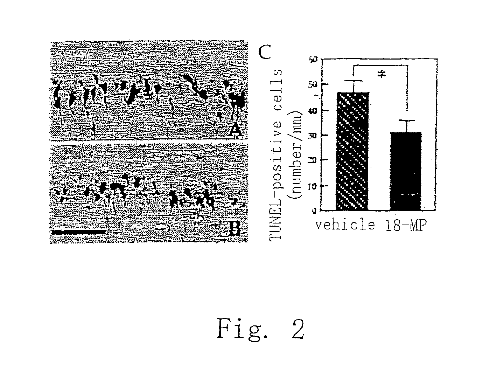

Results are shown in Fig. 2. In Fig. 2, (A) shows TUNEL-positive neurons in the hippocampal CA1 region of a control animal. (B) shows TUNEL-positive neurons in the hippocampal CA1 region of an ischemic animal infused with 18-MP (20 ng/day). As shown in Fig. 2 (A), in the hippocampal CA1 region of the gerbil with 3-minutes transient forebrain ischemia, many TUNEL-positive neurons were noted at 1 week after the ischemic insult, and the neurons were found to be in the course of apoptosis-like neuron death. When continuous 18-MP infusion into the left lateral ventricle was started at 3 days (72 hours) after 3 minutes transient forebrain ischemia, TUNEL-positive neurons were significantly decreased in number as compared with the control ischemic animals [Fig. 2 (B) and Fig. 2 (C)]. This finding indicates that 18-MP suppresses apoptosis-like neuron death in the brain in vivo.

In the above experiment, 18-MP was continuously infused into the left lateral-ventricle starting at 3 days (72 hours) after 3-minutes transient forebrain ischemia. At the initiation of 18-MP infusion (namely at 3 days after 3-minutes transient forebrain ischemia), the hippocampal CA1 pyramidal neurons are not in the hypoxic condition, furthermore they are not be affected by adverse effects of excess oxygen free radicals unlike neurons at the early stage of cerebral ischemia and re-perfusion. Nevertheless, since 18-MP infusion starting at 3 days after 3-minutes transient forebrain ischemia suppressed apoptosis-like neuron death in the hippocampal CA1 field, 18-MP is likely to protect the neurons through a mechanism(s) other than reducing hypoxic injury or neurotoxicity of oxygen free radicals. The activities of 18-MP to reduce hypoxic damage and oxygen free radical damage to neurons have been reported in our previous study (Igase, K. et al., J. Cereb. Blood Flow Metab., 19, 298-306, 1999).

We have further investigated, what would happen to the hippocampal CA1 field without 18-MP infusion from 1 week to 1 year after 3-minutes transient forebrain ischemia. Mongolian gerbils were loaded with 3-minutes forebrain ischemia under inhalation anesthesia according to the method of Sakanaka et al. (Sakanaka, M. et al., Proc. Natl. Acad. Sci. USA, 95, 4635-4640, 1998). Step-down passive avoidance learning experiments were performed at 1 week, 1 month, 3 months, 6 months and 12 months after 3-minutes transient forebrain ischemia. Thereafter the individual animals were perfused transcardially with phosphate buffer containing 4% paraformaldehyde and 2.5% glutaraldehyde under pentobarbital anesthesia. The brains were dissected out and embedded in paraffin, and then paraffin sections 5 µm thick were cut. All neurons with intact morphological oppearance along 1mm linear length of the hippocampal CA1 region were counted in each animal according to the method of Wen et al. (Wen, T.-C. et al., J. Exp. Med., 188, 635-649, 1998). Outlines of the step-down passive avoidance learning experiment are explained as follows (In details, refer to Wen, T.-C. et al., J. Exp. Med., 188, 635-649, 1998; and Sakanaka, M. et al., Proc. Natl. Acad. Sci. USA, 95, 4635-4640, 1998).

One week, one month, 3 months, 6 months and 12 months after 3-minutes transient forebrain ischemia, individual Mongolian gerbils were placed on the safe platform of the passive avoidance learning experiment equipment, but if the gerbils stepped down onto the grid floor, they received foot shock and returned to the safe platform. As a result of 5-minute training, most of the Mongolian gerbils stayed on the safe-platform. 24 hours later, each gerbil was placed on the safe platform while the shock-generator was turned off, and the response latency, i.e., the time until it stepped down onto the grid floor, was measured as an index for learning ability.

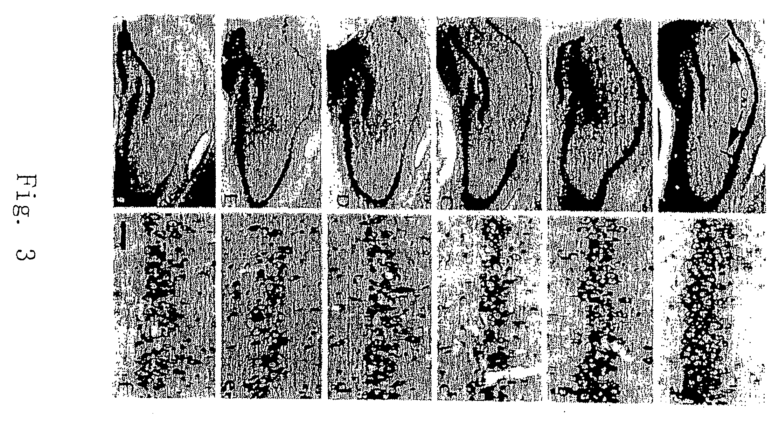

Fig. 3 shows the results of histopathological analysis of the hippocampal CA1 region. Fig. 3A and Fig. 3a show the hippocampal CA1 field of a sham-operated gerbil at low magnification (A) and at high magnification (a). Fig. 3B and Fig. 3b show the hippocampal CA1 field of a gerbil at 1 week after 3-minutes transient forebrain ischemia at low magnification (B) and at high magnification (b). At this time nearly one half of hippocampal CA1 pyramidal neurons degenerated and disappeared. Fig. 3C and Fig. 3c show the hippocampal CA1 field of a gerbil at one month after 3-minutes transient forebrain ischemia at low magnification (C) and at high magnification (c). At this period. The hippocampal CA1 pyramidal neurons decreased further in number. Fig. 3D, Fig. 3d, Fig. 3E, Fig. 3e, Fig. 3F and Fig. 3f, show the hippocampal CA1 field of gerbils at 3 months, 6 months and 12 months (one year) after 3-minutes transient forebrain ischemia at low magnification (D, E, F) and at high magnification (d, e, f), respectively. At these periods, large differences cannot be observed.

Table 1 shows quantified data of the results in Fig. 3 and the response latency of the passive avoidance learning experiment.

**(Table 1)**

| Experimental Group (Time after ischemia) | Neuronal Density (number/mm) | Response Latency. (second) |
|---|---|---|
| Sham operation group | | |
| n=11 | 240.7±4.5 | 239.8±12.5 |
| 1st week | | |
| n=15 | 128.4±7.9² | 119.4±7.4² |
| 1st month | | |
| n=11 | 77.6±8.2^{a,b} | 71.9±6.7^{a,b} |
| 3rd months | | |
| n=7 | 68.3±13.6^{a,b} | 67.9±11.3^{a,b} |
| 6th months | | |
| n=5 | 73.8±29.0^{a,b} | 53.8±13.2^{a,b} |
| 12th months | | |
| n=4 | 71.5±14.4^{a,b} | 31.0±3.7^{a,b,c,d} |
| Test results are shown with mean value ± standard error. | | |
| a, b, c and d mean significant differences from the sham-operated animals and the animals at 1week, 1 month and 3 months after the ischemia respectively, a, b, P<0.01; c, P<0.01; d, P<0.05. | | |
| The atatistical analysis method used is Student's t test with Bonferroni correction for multiple comparison. | | |

Results indicate that the neuronal density in the hippocampal CA1 region reduced to approximately one half at one week after 3-minutes transient forebrain ischemia, and they further decreased in number until one month after ischemia. Thereafter, no significant changes in the neuronal density of the hippocampal CA1 region were noted. Table 1 also shows the response latency in the step-down passive avoidance learning tests in the sham-operated group, and at 7 days (one week), one month, 3 months, 6 months and one year (12 months) after 3-minutes transient forebrain ischemia. The learning ability was significantly lowered at 7 days (1 week) after 3-minutes transient forebrain ischemia as compared with the learning ability of the sham-operated group. One month after 3-minutes forebrain ischemia , the response latency was further significantly reduced. No significant changes in the response latency were noted between 3 months and 6 months after 3-minutes transient forebrain ischemia. One year (12 months) later, in spite of showing no changes in the hippocampal CA1 pyramidal neuron density as compared with that of 6 months, only the response latency was significantly reduced. The reduction of response latency may be due to age-dependent hypofunction of the gerbil brain.

Consequently, in the 3-minutes forebrain ischemia model of Mongolian gerbil, approximately one half of the hippocampal CA1 pyramidal neurons degenerate and disappear at one week after ischemia, and a large number of hippocampal CA1 neurons shows further degeneration within one month after ischemia, then the learning ability may be lowered.

Next, 18-MP was continuously infused into the lateral ventricle of Mongolian gerbils in a dose of 20 ng/day from 3 days to 7 days after 3-minutes transient forebrain ischemia, and the effects of the continuous 18-MP infusion on the hippocampal CA1 pyramidal neuron density and on response latency in the step-down passive avoidance learning experiment were studied. Three minute forebrain ischemia was loaded to Mongolian gerbils under inhalation anesthesia, and 3 days (72 hours) later, 18-MP (20 ng/day) was continuously infused into the left lateral ventricle for 4 days through an osmotic minipump. The step-down passive avoidance learning experiments were conducted as described in our previous studies (Wen, T.-C. et al., J. Exp. Med., 188, 635-649, 1998; and Sakanaka, M. et al., Proc. Natl. Acad. Sci. USA, 95, 4635-4640, 1998). Mongolian gerbils were perfused transcardially with 4% paraformaldehyde and 2.5% glutaraldehyde in phosphate buffer under pentobarbital anesthesia and the brains were dissected out. The brains were embedded in paraffin, paraffin sections 5 µm thick were cut. All neurons with intact morphological oppearance along 1mm linear length of the hippocampal CA1 region were counted according to the method of Wen et al. and Sakanaka et al. (Wen, T.-C. et al., J. Exp. Med., 188, 635-649, 1998; and Sakanaka, M. et al., Proc. Natl. Acad. Sci. USA, 95, 4635-4640, 1998). The equal amount of vehicle (0.01 M phosphate buffered-saline containing 0.1% bovine serum albumin) was infused in the control ischemic animals and in the sham-operated animals.

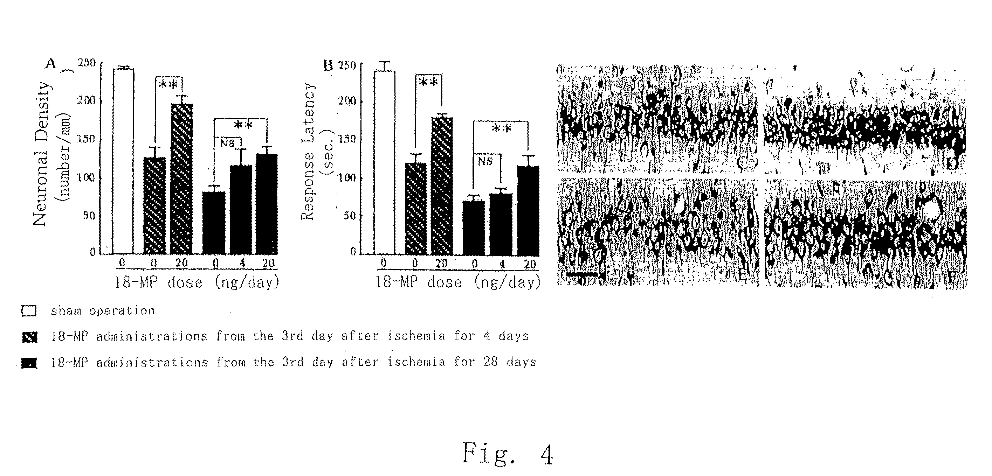

In Fig. 4A, columns with slanting lines show the hippocampal CA1 region of 18-MP-infused ischemic animals and non-infused ischemic animals. In Fig. 4B, columns with slanting lines show response latency of the passive avoidance learning experiments in 18-MP-infused ischemic animals and non-infused ischemic animals. Significant increases in the neuron density and the response latency were observed in animals infused with 18-MP from 3 days to 7 days after 3-minutes transient forebrain ischemia, as compared with non-infused ischemic animals. Fig. 4C and Fig. 4D, show the hippocampal CA1 regions of a non-infused ischemic animal and an ischemic animal with 4 day 18-MP infusion. 18-MP infusion starting at 3 days after 3-minutes forebrain ischemia apparently rescued a large number of hippocampal CA1 pyramidal neurons. In Fig. 4A and Fig. 4B, open columns indicate the experimental results of animals with sham operation. As explained above, only 4 day intracerebroventricular infusion of 18-MP starting at 3 days (72 hours) after 3-minutes transient forebrain ischemia rescued a large number of hippocampal CA1 neurons at one week (7 days) after ischemia. In addition, as shown in Fig. 2, TUNEL-positive neurons at this point were significantly less numerous in the 18-MP administered than in non-administered group. It is plausible that 18-MP infusion prevents degeneration of the hippocampal CA1 neurons, which occurs after one week (7 days) of 3-minutes ischemic insult.

Next, 18-MP was continuously infused into the cerebral ventricle from 3 days to 31 days after 3-minutes transient forebrain ischemia, and the effect of the 28 day 18-MP administration was investigated. Results are shown in Fig. 4A, B, E and F. As shown in the closed columns of Fig. 4A and B. 28 day 18-MP infusion in a dose of 20 ng/day significantly prolonged response latency in the passive avoidance learning exp eriment and rescued a significant number of hippocampal CA1 neurons as compared with the vehicle-infused ischemic group (i.e. 0 ng/day of 18-MP-infused group). Fig. 4E shows a photomicrograph of the hippocampal CA1 region of an ischemic animal infused with vehicle from 3 days (72 hours) to 31 days after 3-minutes transient forebrain ischemia and Fig. 4F shows a photomicrograph of the hippocampal CA1 region of an ischemic animal infused with 18-MP (20 ng/day) in the same schedule. Therefore, a significant number of hippocampal CA1 pyramidal neurons was rescued by the 28 day administration of 18-MP (20 ng/day) which started at 3 days after 3-minutes transient forebrain ischemia. Administration of 18-MP in a dose of 4 ng/day showed no significant effect. In Fig. 4A and B, NS means no significant differences. As shown in the above, since the 3-minutes forebrain ischemia model of Mongolian gerbil exhibits long term neuron death in the hippocampal CA1 region, it is specifically useful for the screening of neuroprotective agents Apparently, the present ischemia model shows similar phenomena to the slowly progressive neuron death observed in neurodegerative diseases and after a mild transient ischemic attack or carbon monoxide poisoning.

From 3 days to 31 days after 3-minutes transient forebrain ischemia, the hippocampal CA1 region is not generally exposed to hypoxic condition or to excess oxygen free radicals. Consequently, the fact that 18-MP suppresses apoptosis-like neuron death in the hippocampal CA1 region during 3-31 days after ischemia, indicate that 18-MP protects neurons through a mechanism(s) apart from the hypoxic damage-reducing action and the oxygen free radical damage-reducing action, as described in the explanation in Fig. 2. Namely, the hypothesis that 18-MP protects neurons by reducing the hypoxic and free radical injuries, as proposed by the inventors of the present invention and Igase et al. (Igase, K. et al., J. Cereb. Blood Flow Metab., 19, 298-306, 1999), is not always pertinent. If the detailed action mechanism of 18-MP is elucidated, novel effect and efficacy of 18-MP may be discovered, and thus an action mechanism of 18-MP was analyzed by using cultured experimental systems.

As reported in the U.S. Patents (No. 5,571,787, Prosaposin as a neurotrophic factor; U.S. Patent NO. 5,696,080, Pharmaceutical compositions comprising neurotrophic peptide derived from prosaposin; U.S. Patent No. 5,700,909, Prosaposin and cytokine-derived peptides; U.S. Patent No. 5,714,459, Use of prosaposin and neurotrophic peptides derived therefrom), 18-MP promotes neurite outgrowth of NS20Y neuroblastoma cells at high concentration levels of 0.01 - 0.5 µg/ml, but actions of 18-MP at the lower levels are not elucidated except for preventing neuronal necrosis caused by ferrous sulfate (hydroxylradical inducer)(Igase, K. et al., J. Cereb. Blood Flow Metab., 19, 298-306, 1999). Accordingly, we have investigated whether or not low concentrations of 18-MP suppress apoptosis-like neuron death or apoptosis of neurons, using primary neuron cultures.

Cell death is mainly classified into necrosis and apoptosis based on its morphological features. With regard to neuron death, the concept of necrosis has been established, but the concept of apoptosis has not been fully established. Similar phenomena to apoptosis of lymphocytes are observed in the pathological mature brain, but typical morphological features as seen in lymphocytes in the course of apoptosis are rarely noted. Consequently, in the present specification, slowly progressing neuron death, which is different from necrosis, is defined as apoptosis of neurons or apoptosis-like neuron death, as described hereinbefore.

We (Sakanaka and Tanaka) have recently found that apoptosis of neurons or apotosis-like neuron death is induced by exposing cultured neurons to sodium nitroprus-side (SNP), a nitric oxide (NO) donor, for a short period (Toku, K. et al., J. Neurosci. Res., 53, 415-425, 1998). Using this experimental system, we investigated the suppressive effect of 18-MP on apoptosis-like neuron death.

Neurons from the cerebral cortices of 17-day-old rat embryos were incubated in a serum-free medium for 4 or 5 days, and the medium was replace with the fresh medium-with or without 18-MP, and the neurons were further cultured for 24 hours. SNP at a concentration of 100 µM was added to the medium for 10 minutes. Then the neurons were incubated in the medium containing 18-MP and survival-rate of the neurons was measured with the use of a redox indicator, alamer blue, according to the method of Toku et al. (Toku, K. et al., J. Neurosci. Res., 53, 415-425, 1998).

In the report of the present inventors and Igase et al. (Igase, K. et al., J. Cereb. Blood Flow Metab., 19, 298-306, 1999), the effect of 18-MP on oxygen-free radical injury to neurons was determined by adding 18-MP to the culture medium prior to treatment with the hydroxylradical inducer (ferrous-sulfate), however in the present experiment, 18-MP was added to the culture medium-before and/or after SNP treatment.

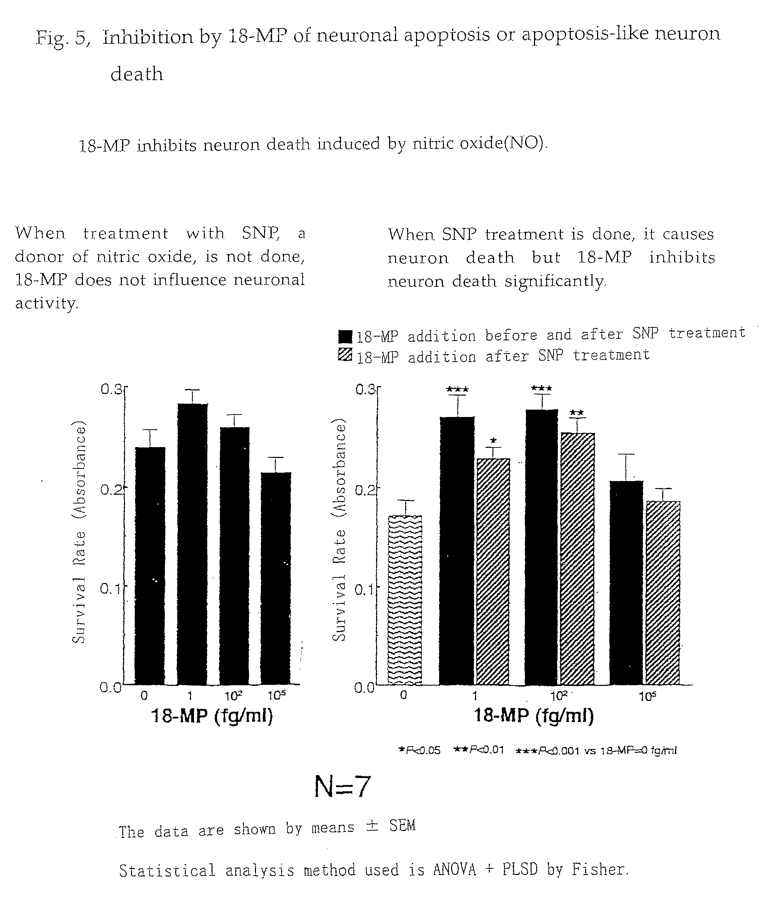

Results are shown in Fig. 5. Closed columns on the left side of Fig. 5 indicate the survival rate of neurons with or without addition of 18-MP without SNP treatment. Closed columns on the right side of Fig. 5 indicate the survival rate of neurons with addition of 18-MP before and after SNP treatment. Columns with slanting lines on the right side of Fig. 5 indicate the survival rate of neurons with addition of 18-MP after SNP treatment. As shown on the left side of Fig. 5, without SNP treatment, 18-MP did not significantly affect the metabolic activity of cultured neurons. When neurons were treated with only SNP without addition of 18-MP, apoptosis-like neuron death or apoptosis of neurons occurred as shown in the column with wave lines on the right side of Fig. 5. 18-MP significantly suppressed, even at the concentration range of 1 - 100 fg/ml, apoptosis-like neuron death (apoptosis of neurons) not only in the case of 18-MP addition before and after SNP treatment but also in the case of 18-MP addition after SNP treatment. However, 18-MP at such a low concentration range did not facilitate neurite outgrowth, different from the effect of high concentrations (0.01 - 0.5 µg/ml) of 18-MP on neurite elongation. As for 18-MP, promoting neurite outgrowth and extending neuron survival may be discussed separately. Neurite elongation induced by 18-MP at the high concentrations (0.01 - 0.5 µg/ml), which is reported in the U.S. Patents (No. 5,571,787, Prosaposin as a neurotrophic factor; U.S. Patent No. 5,696,080, Pharmaceutical compositions comprising neurotrophic peptide derived from prosaposin; U.S. Patent No. 5,700,909, Prosaposin and cytokine-derived peptides; U.S. Patent No. 5,714,459, Use of prosaposin and neurotrophic peptides derived therefrom), may not always suggest suppression of neuron death by the high concentrations of 18-MP. Namely, in the said U.S. Patents, 18-MP at the high concentrations were expected to suppress neuron death, and the possibility of the high concentrations of 18-MP to apply for prevention, therapy and treatment of cerebral stroke and neurodegenerative diseases was raised. On the contrary, the present invention newly discloses that low-concentrations of 18-MP prevent and treat apoptosis-like neuron death or apoptosis of neurons. The present finding that 18-MP at 1-100 fg/ml suppresses apoptosis-like neuron death or apoptosis of neurons proves for the first time in the world as a pioneer that low extracellular levels of 18-MP can be applied for prevention, therapy and treatment of pathogenic apoptosis-like neuron death.

The suppressive action of the low concentrations of 18-MP on apoptosis-like neuron death is similar to that of ginsenoside Rb₁, which is described in Japanese Patent Application No. Hei 10-365560 (Protective agents for brain cells or nerve cells comprising ginsenoside Rb₁). Ginsenoside Rb₁ is a non-peptide substance consisting of a steroid-like structure with 4 molecules of sugar chains and it stimulates the expression of Bcl-x_{L}. Although ginsenoside Rb₁ has no common chemical structure with 18-MP, we noted the similar physiological actions of both substances, and investigated whether the low concentrations of 18-MP induces the expression of the cell death suppressing gene product, i.e. Bcl-x_{L} protein, as does ginsenoside Rb₁. Bcl-x_{L} gene product is expressed in almost all tissues such as the skin, brain and the other tissues in the immune, digestive, circulation, respiratory, endocrine, urogenital and musculoskeletal systems and it plays important roles in the survival of cells (Adams, J.M. and Cory, S. Science, 281, 1322-1326, 1998; Boise, L.H. et al., Cell, 74, 597-608, 1993; Gottschalk, A.R. et al., Proc. Natl. Acad. Sci. USA, 91, 7350-7354, 1994; Gonzaletz-Garcia, M. et al., Proc. Natl. Acad. Sci. USA, 92, 4304-4308, 1995).

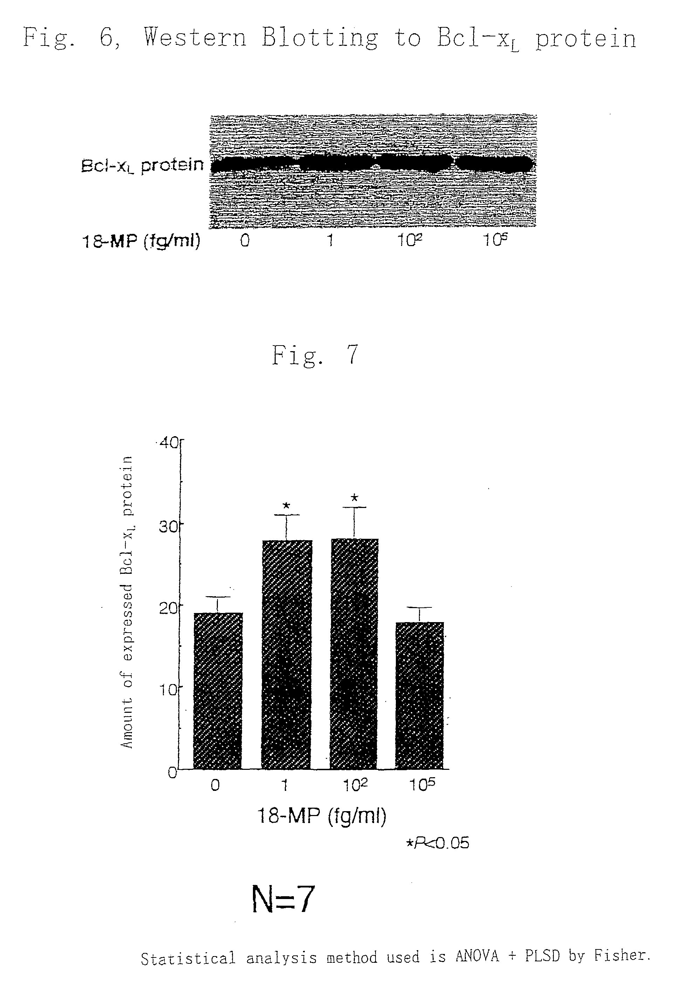

In order to investigate whether the low concentrations of 18-MP enhance the expression of Bcl-x_{L} protein in nerve cells or neurons, the western blotting technique was applied with the use of anti-Bcl-x_{L} protein antibody. Neurons from the cerebral cortices of 17-day-old rat embryos were cultured for 48 hours with or without the low concentrations of 18-MP. The neurons were lysed in sample buffer for electrophoresis and electrophoresed. Then the electrophoretic proteins were transferred to nitrocellulose membrane and subjected to western blotting. Results are shown in Fig. 6.

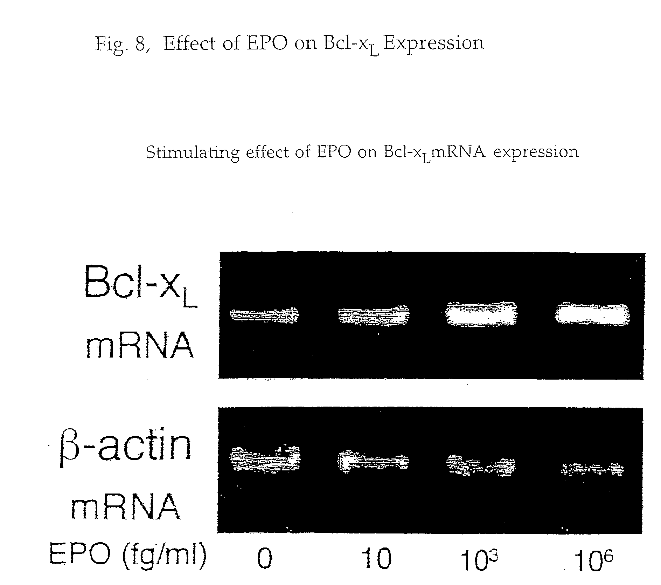

Further, bands reacted with the anti-Bcl-x_{L} protein antibody were quantified by image analysis. Results are shown in Fig. 7.

As shown in Fig. 6 and Fig. 7, 18-MP significantly increased the expression of Bcl-x_{L} protein in neurons within the concentration range of about 1 - 100 fg/ml. In the same concentration range, 18-MP suppresses apoptosis-like neuron death as shown in Fig. 5.

As for peptide bioactive substances that enhance the expression of Bcl-x_{L} protein in neurons, we (Sakanaka, Tanaka and Satoh) have reported interleukin 3 at the extracellular levels of 0.6 - 15.0 ng/ml (Wen, T.-C. et al., J. Exp. Med., 188, 635-649, 1998). The concentrations of 18-MP to stimulate Bcl-x_{L} protein expression are far lower than those of interleukin 3, and 18-MP is a more potent inducer of Bcl-x_{L} protein expression than interleukin 3, which can increase the expression of Bcl-x_{L} protein in neurons only by approximately 10%. The stimulating action of the low concentrations of 18-MP on Bcl-x_{L} protein expression is almost equal to that of ginsenoside Rb₁, which is disclosed in Japanese Patent Application No. Hei 10-365560 and PCT/JP99/02550 (Protective agents for brain cells or neurons comprising ginsenoside Rb₁). According to Banks et al., (Banks, W. et al., Peptides, 13, 1289-1294, 1992), radioactive iodine-labeled human 18-MP, in which valine residue at No. 12 amino acid from N-terminal of saposin C domain is replaced with tyrosine residue (molecular weight approximately 2000), enters the brain through the blood-brain barrier (BBB) after intravenous administration. Consequently, prosaposin-related 18-mer peptides derived from various animals having consensus amino acid sequence (LIXNNX, wherein X is any one of amino acids) as shown in Fig. 1, or peptides constituted by amino acids smaller in number than that and having the same consensus amino acid sequence, have a high possibility to enter the brain parenchyma passing through the BBB after intravenous administration. As described in the prior arts of the present specification, peptides having the hereinbefore described consensus sequence (consensus amino acid sequence) are defined totally as 18-MP in the present specification. In the present experiments, although data obtained by using rat 18-MP are presented, the biological actions of rat 18-MP appear to be applied to all of peptides having the above consensus sequence (consensus amino acid sequence). In fact, it is known that neurite outgrowth-promoting action is exhibited in the high concentration range (0.01 - 0.5 µg/ml) common to human 18-MP and rat 18-MP (U.S. Patents No. 5,571,787, Prosaposin as a neurotrophic factor; U.S. Patent No. 5,696,080, Pharmaceutical compositions comprising neurotrophic peptide derived from prosaposin; U.S. Patent No. 5,700,909, Prosaposin and cytokine-derived peptides; U.S. Patent No. 5,714,459, Use of prosaposin and neurotrophic peptides derived therefrom; and Kotani, Y. et al., J. Neurochem. 66, 2197-2200, 1996). Further, the other compounds containing any peptides comprising the consensus sequence (consensus amino acid sequence) hereinbefore are likely to show the same biological actions, effects and efficacy as those of rat 18-MP.

Among peptides stimulating Bcl-x_{L} protein expression in neurons, although interleukin 3 can not show protective action on neurons unless directly administered intracerebroventricularly, 18-MP has the possibility to protect brain cells, glial cells or neurons even by intravenous administration. If 18-MP is stable in the digestive tract, its oral administration may be possible. Oral administration of 18-MP is possible by mixing, encapsulating or binding of 18-MP with any carriers which suppress decomposition or degradation of 18-MP in the digestive tract.

The mitochondria-associated protein Bcl-x_{L} is bound with Apaf1 to inhibit interaction between Apaf1 and procaspase 9 (Adams, J.M. and Cory, S. Science, 281, 1322-1326, 1998). Once decrease in Bcl-x_{L} protein or hypofunction of Bcl-X_{L} protein takes place, Apaf1 is released from Bcl-x_{L} to bind with procaspase 9, thereby activating procaspase 9 in harmony with the leakage of cytochrome C from mitochondria. The activation of procaspase 9 in the cytoplasm leads to the activation of caspase 9 and caspase 3 and the autolysis of cells occurs by the actions of these proteolytic enzymes. This event is termed apoptosis or apoptosis-like cell death. Since cells are destined to enter apoptosis or apoptosis-like death in response to the activation of procaspase 9, the suppression of procaspase 9 activation by 18-MP, which enhances Bcl-x_{L} protein expression, appears to be beneficial for the therapy, treatment or prevention of diseases involving cell death.

Next, we have searched for peptides or proteins enhancing Bcl-x_{L} protein expression in neurons, besides interleukin 3 and 18-MP. In this connection, we have focused on a cytokine, i.e. erythropoietin (EPO), which suppresses delayed neuronal death in the hippocampal CA1 field of Mongolian gerbil as do interleukin 3 and 18-MP and is included within the category of colony-stimulating factors similar to interleukin 3. EPO is applied for the treatment of renal anemia as a cytokine to stimulate erythropoiesis, and EPO is produced not only in the kidneys but also in the glial cells of brain (as torcytes)(Masuda, S. et al., J. Biol. Chem., 269, 19488-19493, 1994). In addition, EPO receptor is expressed in neurons or nerve cells (Morishita, E. et al., Neuroscience, 76, 105-116, 1997; Morishita, E. et al., Blood, 88, 465-471, 1996). Consequently, binding of EPO with EPO receptor occurs not only in the bone marrow but also in the brain, thereby playing important physiological roles. One of the inventors of the present invention (Sakanaka) reported that EPO significantly reduced learning disability and hippocampal CA1 neuron death of Mongolian gerbil which was loaded with 3-minutes transient forebrain ischemia (Sakanaka, M. et al., Proc. Natl. Acad. Sci. USA, 95, 4635-4640, 1998). However, what type of survival factor is expressed in cells as a result of binding of EPO with EPO receptor on the neuronal cell membrane, is still unknown. Consequently, we have investigated whether EPO could enhance Bcl-x_{L} gene expression as did interleukin 3.

Experimental techniques are the same as those described in the study of the inventors (Sakanaka, Tanaka and Satoh) and Wen et al. (Wen, T.-C. et al., J. Exp. Med., 188, 635-649, 1998). Neurons from cerebral cortices of 17-day-old rat embryos were cultured in a medium containing 10% fetal calf serum. On the 2nd day of culture, the medium was replaced with a serum-free medium, and on the 4th day of culture, EPO (0, 10, 10³, or 10⁶ fg/ml) was added to the medium, then the neurons were cultured for 24 hours. Thereafter total RNA was extracted from the cultured neurons. cDNA was prepared from DNAase-treated total RNA (3 µg) by using oligo dT primer and reverse transcriptase. PCR was conducted with the use of Taq polymerase in the following condition, i.e. 20 cycles (for -actin) or 25 cycles (for Bcl-x_{L}) at (1) with 94°C, 2 min.; (2) a cycle with 94°C, 1.5 min.; 55°C, 1.5 min.; 72°C, 2 min.

The PCR products were electrophoresed on 3% agarose gel and visualized with ethidium bromide. Expression of -actin mRNA was used as an internal standard. Results are shown in Fig. 8. As shown in Fig. 8, the expression of Bcl-x_{L} mRNA was markedly enhanced in neurons treated with EPO (10³ and 10⁶ fg/ml) as compared with neurons without EPO treatment. Especially, in neurons treated with 10³ fg/ml of EPO, the expression of Bcl-x_{L} mRNA was markedly increased. Since the molecular weight of EPO (molecular weight approximately 30Kd) is larger than that of 18-MP, 18-MP and EPO appear to enhance the expression of Bcl-x_{L} at almost equal molar concentrations.

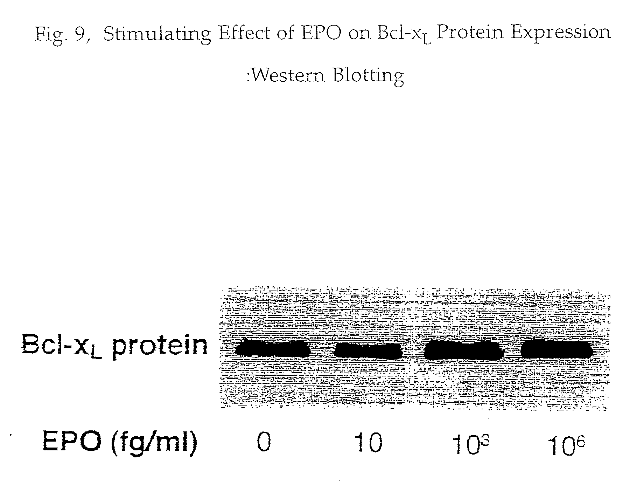

We have further investigated whether EPO could enhance the expression of Bcl-x_{L} protein in primary cultured neurons. Neurons from the cerebral cortices of 17-day-old rat embryos were cultured for 48 hours with EPO (10, 10³, or 10⁶ fg/ml) or without EPO. The neurons were lysed with sample buffer for electrophoresis and subjected to electrophoresis. Electrophoresed proteins were transferred to nitrocellulose membrane and western blotting with the anti-Bcl-x_{L} antibody was performed. Results are shown in Fig. 9. As shown in Fig. 9, the expression of Bcl-x_{L} protein is increased in the neurons treated with EPO at concentrations of 10³ and 10⁶ fg/ml as compared with neurons without EPO treatment, and especially at the concentration of 10³ fg/ml, EPO stimulated Bcl-x_{L} protein expression the most effectively. The effect of EPO at a concentration 10 fg/ml on Bcl-x_{L} protein expression was not clear, although EPO at the same concentration seemed to upregulate the expression of Bcl-x_{L} mRNA.

In the U.S. Patent (No. 5,700,909, Prosaposin and cytokine-derived peptides), it is proposed that partial amino acid sequences of series of peptide growth factors and cytokines shown in Table 2 promote neurite outgrowth at the concentration of 10 ng/ml or more as do the partial amino acid sequences (including 18-MP) of saposin C.

**(Table 2)**

| Cytokine | Peptide | Existing Site |
|---|---|---|
| human ciliary neurotiophic factor (hCNTF) | YVKHQGLNKNINLDSVDGVT | Loop AB |
| human interleukin6(hIL-6) | EALAENNLNLPKMAG | Loop AB |
| human interleukin2 (hIL-2) | LQMILNGINNYKNPKLT | Loop AB |
| human interleukin3 (hIL-3) | ILMENNLRRPNL | Loop AB |
| human interleukin1 γ (hIL-1γ) | FYLRNNQLVAGTL | Loop AB |
| human erythropoietin (hEPO) | AEHCSLNENTTVPDTKU | Loop AB |
| human leukemia inhibitory factor (hLIF) | YTAQGEPFPNNVELKLCAP | Loop AB |
| human interleukin1 β (hIL-1 β) | FNKIEINNKLEFESA | Helix C |
| human ocostatinM (hONC-M) | RPNILGLRNNTYCMAQLL | Helix C |

The U.S. Patent (No. 5,700,909, Prosaposin and cytokine-derived peptides) describes that these partial amino acid sequences, i.e. fragments, rat prosaposin-related peptide (LSELIINNATEELLIKGL) used in the present example, and the human prosaposin-related peptide disclosed in the above U.S. Patent (No. 5,700,909, Prosaposin and cytokine-derived peptides) (CEFLVKEVTKLIDNNKTEKEIL) have the consensus amino acid sequence, i.e. xNNyz. The most important features of the said consensus sequence are: (1) to have two continued asparagine (N) residues or to have any one of amino acids between two asparagine residues, (2) to have x, i.e. the moety containing leucine (L) residue or isoleucine (I) residue, which is 3 or 4 residues distant from two asparagine residues towards the N-terminus of said asparagine residues , (3) to have one or more charged amino acid residues y, i.e. aspartic acid (D), lysine (K), glutamic acid (E) or arginine (R), which is located 2 to 8 residues towards the C-terminus of said asparagine residues, and (4) to have one or more hydrophobic amino acid residues z, i.e. alanine (A), leucine (L), isoleucine (I) or valine (V), which is located 6 to 10 residues towards the C-terminus of said asparagine residues. 18-MP used in the experiment in this specification, i.e. LSELIINNATEELLIKGL, human CNTF (ciliary neurotrophic factor, hCNTF) fragment, human interleukin 2 (hIL-2) fragment, human interleukin 3 (hIL-3) fragment, and human interleukin 1- (hIL 1-) fragment have the above specific features. Human EPO (hEPO) fragment and human leukemia inhibitory factor (hLIF) fragment do not have the specific feature of (2). Human interleukin 1- (hIL 1-) fragment and human oncostatin M (hONC-M) fragment do not exist in AB loop but exist in helix C. The U.S. Patent (No. 5,700,909, Prosaposin and cytokine-derived peptides) assumes that these peptide fragments described in Table 2 have all common biological activity.

Since 18-MP used in the example in the present specification, i.e. LSELIINNATEELLIKGL, has the specific feature of the above described xNNyz, the fact that the low conc entrations of 18-MP suppresses apoptosis-like neuron death and enhances the expression of Bcl-x_{L}, indicates that the peptide fragments described in Table 2 suppress apoptosis-like neuron death and stimulate the expression of Bcl-x_{L} in the same low concentration range as that of 18-MP. Proviso that since we have reported that interleukin 3 induced the expression of Bcl-x_{L} in the concentration range from 0.6 ng/ml to 15.0 ng/ml (Wen, T.-C. et al., J. Exp. Med., 188, 635-649, 1998), hIL-3 fragment in Table 2 may enhance the expression of Bcl-x_{L} protein even at high concentration. Further, the experimental result in Fig. 9, in which EPO (hEPO) enhanced the expression of Bcl-x_{L} protein at the same molarity as 18-MP, supports the view that hEPO-fragment having the amino acid sequence common to 18-MP induces Bcl-x_{L} expression at the similar low molarity. Moreover, all cytokines, chemokines, growth factors and non-peptide agents like ginsenoside Rb1 as well as their metabolites or fragments with neuroprotective activity in vivo are likely to facilitate cell survival in peripheral tissues and organs at the low molarity similar to 18-MP by regulating the expression fo Bcl-x_{L} protein and/or the other Bcl-2 family proteins.

Next, we have investigated that 18-MP protected not only neurons but also cells in peripheral tissues, and enhanced the expression of Bcl-x_{L} protein in peripheral tissues. To this end, experiments were performed by using, for example, cultured cardiac myocytes.

Hearts were dissected out fromn 17-day-old rat embryos and the atrium was removed. The tissues were cut into small pieces with scissors, and incubated with trypsin-EDTA solution at room temperature for one hour to disperse cells. In order to remove contaminated fibroblasts, the cell suspension was put into conventional dishes for cell culture and incubated for 20 minutes. Non-adhered cells were seeded on a 24 well plate for primary culture (Falcon Inc.) and cultured in DMEM (Dulbecco's modified Eagle's medium) containing 10% FCS (fetal calf serum).

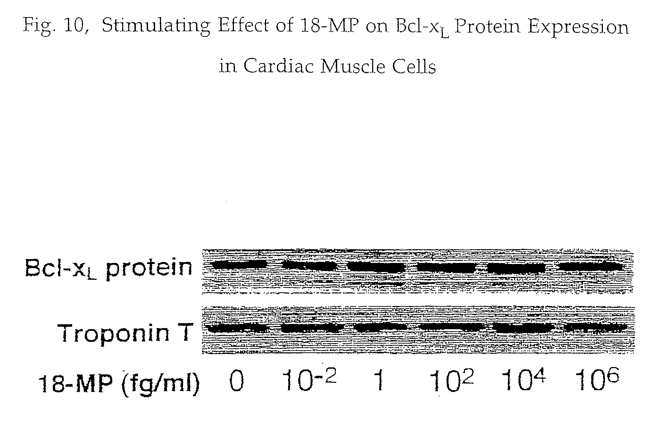

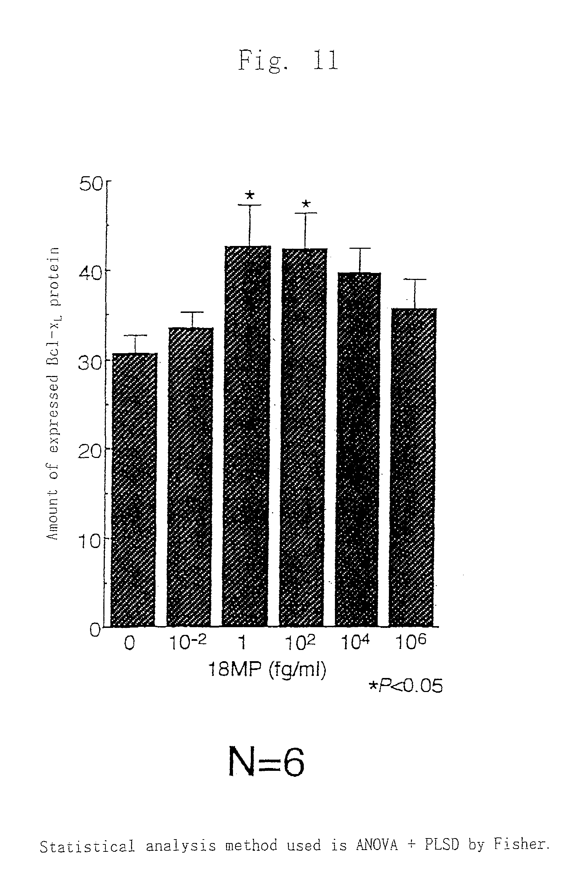

18-MP was added to the cultured cardiac myocytes on the 24 well plate at 0.01 fg/ml - 1 ng/ml. 24 hours later, homogenates were prepared, electrophoresed and subjected to western blotting by using the anti-Bcl-x_{L} antibody. Troponin T, which was only expressed in the striated muscle, was used as an internal standard. Bands reacted with the anti-Bcl-x_{L} antibody were quantified with an image analyzer. In addition, cultured cardiac myocytes in the 24 well plate were maintained in a culture medium devoid of glucose, to which 18-MP was added at the concentrations of 0.01 fg/ml - 1 ng/ml. After cultured for 4 - 5 days, the cardiac muscle cells (myocytes) in each well were lysed and subjected to western blotting with an antibody against muscle-apecific α actinin to estimate the survival rates of cardiac muscle cells that were treated with different concentrations of 18-MP. Results are shown in Fig. 10, Fig. 11 and Fig. 12.

As shown in Fig. 10 and Fig. 11, 18-MP, in the concentration range between 1 fg/ml and 100 fg/ml, significantly enhanced the expression of Bcl-x_{L} protein in cardiac muscle cells (myocytes). Since there was no change in the amount of troponin T detected in each well of the 24 well plate, 18-MP appeared to stimulate the expression of Bcl-x_{L} protein without affecting the number or survival rate of cardiac muscle cells (myocytes) during the 24 hour cultivation.

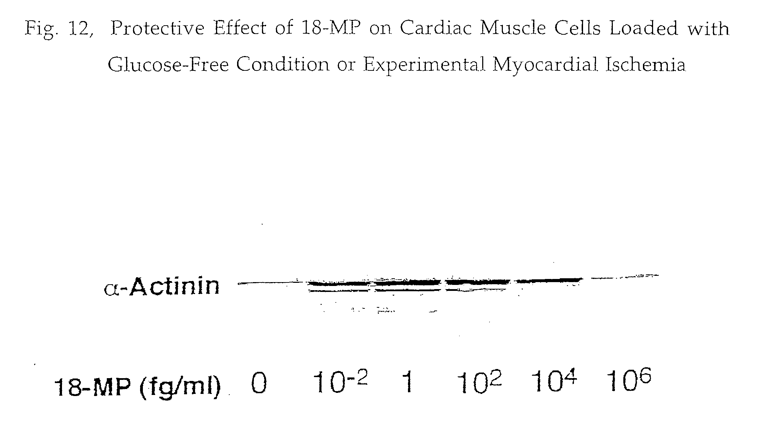

As shown in Fig. 12, 4-5 day treatment of cardiac muscle cells in a glucose-free medium with 18-MP at concentrations of 10⁻²-10⁴ fg/ml apparently suppressed a decline in the amount of striated muscle-specific α-actinin as detected by western blotting. When cultured only with the glucose-free medium devoid of 18-MP, cardiac muscle cells almost disappeared within 4-5 days. On the contrary, large numbers of cardiac muscle cells (myocytes) were observed in the wells treated with 18-MP at concentrations of 0.01 fg/ml, 1 fg/ml, 100 fg/ml and 10 pg/ml. Further in the presence of a higher concentration of 18-MP(10⁶ fg/ml), the survival of cardiac myocytes was not facilitated. Thus, apoptosis-like cardiac muscle cell death was significantly suppressed by adding the low concentrations of 18-MP to the culture medium devoid of glucose. Consequently, 18-MP exhibits protective action on cardiac myocytes in the wide extracellular concentration range as compared with the effective concentration range of 18-MP for neurons. Since 18-MP exhibits protective action on cardiac myocytes in a wider concentration range than the concentration range for enhancing the expression of Bcl-x_{L} protein, 18-MP may suppress apoptosis or apoptosis-like cell death in the cardiac muscle by regulating functions of the other cell death related-factors such as Bcl-2, Bcl-w, Bax, Bad, Bak, Bik, Fas and caspases, in addition to promoting Bcl-x_{L} protein expression in cardiac myocytes. However, on the basis of the results shown in Fig. 5, Fig. 6, Fig. 7, Fig. 10, Fig. 11 and Fig. 12, the enhancement of Bcl-x_{L} protein expression appears to be a central mechanism underling the cytoprotective action of 18-MP. The above findings indicate the effectiveness of 18-MP on prevention, therapy or treatment of the peripheral tissue diseases involving cell death.

As a result of the above experiments, 18-MP at the low concentrations, i.e. extracellular levels in the lesion tissue or the local region at 1 ng/ml (approximately 0.5 nM) or less, preferably 10 pg/ml (approximately 5 pM) or less, more preferably 1 - 100 fg/ml (about 0.5 - 50 fM), promotes the expression of cell death-suppressing gene product Bcl-x_{L}. Further, the peptide fragments mentioned in Table 2 appear to have the same action as that of 18-MP.

18-MP used in the present invention is known as a partial peptide of prosaposin which is contained in human milk, brain and cerebrospinal fluid, and is a substance with few ill effects.

Accordingly, the present invention provides clinically useful cytoprotective agents and method for treatment. Therapeutic, preventive or treatment agents or methods for brain diseases and peripheral tissue diseases of the present invention are prepared so that the extracellular fluid levels of 18-MP in lesion tissue are at 1 ng/ml (approximately 0.5 nM) or less, preferably at 10 pg/ml (approximately 5 pM) or less, and more preferably at 1 - 100 fg/ml (approximately 0.5 - 50 fM). More particularly, the present invention provides agents for therapy, prevention or treatment of brain and peripheral tissue diseases and agents for protection of any types of cells, comprising preparation for intravenous administration adjusted in the extracellular levels of 18MP at 1 ng/ml (approximately 0.5 nM) or less, preferably at 10 pg/ml (approximately 5 pM) or less, more preferably at 1 - 100 fg/ml (approximately 0.5 - 50 fM). The peptide fragments mentioned in Table 2 may also be used as the same pharmaceutical preparations as those of 18-MP.

An agent or a method for therapy, prevention or treatment of brain diseases and peripheral tissue diseases in the present invention can be used in the form of infusing agent for local lesion, agent for external use, suppository, nasal drops, ear drops, inhalation, sublingual agent, eye ointment and eye drops, if the extracellular concentrations of 18-MP in the lesion tissue can be kept low as described hereinbefore.

The pharmaceutical composition comprising 18-MP of the present invention enhances the expression of Bcl-x_{L} protein at the low concentrations and is effective for brain diseases accompanied by apoptosis-like neuron death (cerebral apoplexy, stroke, Alzheimer's disease, Pick's disease; spinocerebellar degeneration, Parkinson's disease, chorea, amyotrophic lateral sclerosis, AIDS encephalopathy, hepatic encephalopathy, progressive supranuclear palsy, multiple sclerosis, demyelinating diseases, encephalitis, cerebral palsy, head injury, spinal cord injury, neonatal asphyxia, peripheral nerve damage, alcoholism, vitamine deficiency, intracranial hypertension, brain edema, prion disease, hydrocephalus, epilepsy, arteriovenous malformation, ischemic leukoencephalopathy, Shy-Drager syndrome). The effective extracellular concentrations of 18-MP in lesion tissue, i.e. concentrations to enhance the expression of Bcl-x_{L} is far lower than the previously reported concentrations for promoting neurite outgrowth in the following U.S. Patents (U.S. Patent No. 5,571,787, Prosaposin as a neurotrophic factor; U.S. Patent No. 5,696,080, Pharmaceutical compositions comprising neurotrophic peptide derived from prosaposin; U.S. Patent No. 5,700,909, Prosaposin and cytokine-derived peptides; U.S. Patent No. 5,714,459, Use of prosaposin and neurotrophic peptides derived therefrom), and this is a specific feature of the present invention.

The pharmaceutical composition of the present invention has almost no adverse drug reactions and the present invention provides drug with high safety.

The present invention discloses that 18-MP suppresses apoptosis or apoptosis-like cell death at the unprecedentedly low extracellular concentrations among the prior known peptide factors by promoting Bcl-x_{L} protein expression. This indicates the effectiveness of 18-MP for not only central nervous system diseases but also peripheral tissue diseases accompanied by apoptosis or apoptosis-like cell death (for example, contact dermatitis, hand and finger dermatitis, senile xerosis, asteatotic dermatitis, nummular eczema, autosensitization dermatitis, seborrheic dermatitis, dundruff, erythroderma, generalized exfoliative dermatitis, urticaria, cutaneous pruritus, insect bite, strophulus, prurigo, purpura, progressive pigmented purpuric dermatitis, Shamberg disease, erythema exsudativum multiforme, annular erythema, erythema nodosum, pernio, chilblain, drug eruption, photosensitivity, epidermolysis bullosa, pemphigus, bullous pemphigoid, dermatitis herpetiformis, pustulosis palmaris et plantaris, psoriasis, lichen planus, ichthyosis, lichen pilaris xanthomatosis, cutaneous amyloidosis, herpes simplex, herpes zoster, viral wart, molluscum contagiosum, pyoderma, skin tuberculosis, atypical mycobacteriosis of the skin, tinea, cutaneous candidiasis, oral candidiasis, scabies, pediculosis pubis, syphilis, keloid, hypertrophic scar, angioma, lymphangioma, vitiligo vulgaris, ephelides, chloasma, melanosis, Pompholyx, miliaria, osmidrosis, acne, rosacea, rosacea-like dermatitis, perioral dermatitis, alopecia, leg ulcer, rejection after organ transplantation, ischemia re-perfusion injury of cardiac muscle, liver and kidneys, alcohlic liver disease, myocardial infarction, drug-induced liver injury, hepatitis, nephritis, fatty liver, burn, radiation injury, ultraviolet ray injury, cardiac insufficiency, cardiomyopathy, occlusion of peripheral arteries, thromboangitis obliterans, arteriosclerosis, atherosclerosis, arteriosclerosis obliterans, silent myocardial ischemia, angina pectoris, diabetic retinopathy, thrombophrebitis, heart failure, peripheral circulation insufficiency, atopic dermatitis, AIDS, bedsore, corneal injury, renal failure, hepatic failure, autoimmune diseases, postoperational wound, diabetic nephropathy, diabetic angiopathy, traumatic wound, non-traumatic wound, shock, skin injury, collagen diseases, immunodeficiency disease, peptic ulcer and diabetes mellitus, aplastic anemia, myelodysplastic syndrome, leukopenia, agranulocytosis, thrombocytopenia, anemia and so on). It is likely that 18-MP is useful for the therapy, prevention or treatment of any organic diseases involving cell death, except psychiatric disorders such as schizophrenia and depression. The other cell death-mediated diseases or pathologic conditions not mentioned in this speification are described in a book entitled Today's Therapy 1995 (edited by Inagaki, Y., Takasu, Y, Ogata, E., under the supervision of Hinohara, S., Abe, M., Igaku shoin Ltd, Tokyo, 1995, PP 17-34). All the above diseases or pathologic conditions can be treated, cured or prevented by the pharmaceutical composition comprising 18-MP. The pharmaceutical composition comprising 18-MP is apparently applied to domestic animals and pets suffering from the cell death-mediated diseases and/or pathologic conditions mentioned above. Further, the low concentrations of 18-MP appear to be effective for the protection and maintenance of blood cell compositions and platelet for transfusion, for the protection and maintenance of frozen or non-frozen ovum, stem cells, ES cells or sperms, and for the protection and maintenance of frozen or non-frozen keratinocyte sheets cultured for skin grafting. In the other organs for transplantation (liver, kidney, heart, pancreas, lung, digestive tract, cornea, blood vessel, and others), the organs can be dipped in or perfused with the solution of low concentrations of 18-MP during, before or after the transplantation, and thereby the organs can be protected to make the transplantation successful.

The symptoms of ageing of the skin include atrophy, wrinkling, chap, sagging, laxity, dryness, vulnerability to infection, exfoliation, itching, gray hair, alopecia and mottled pigmentation. These symptoms are elicited by age-dependent hypofunction, decrease, loss or death of cells in the skin and/or by ultraviolet ray-mediated damage to cells in the skin. 18-MP with a potent cytoprotective action can be used as an ingredient or a composition of any cosmetics to prevent or improve the symptoms or signs of ageing of the skin. 18-MP at the low concentrations can be also contained in any cosmetics or external preparations for chemical peeling of the skin. 18-MP can be used as a cosmetic composition together with ginsenosides, ginseng soponins, ginseng extract, methyl parahydroxybenzoate, emulsifying excipient qsp, alkyl glucoside, butylhydroxyanisole, cepharantine, Eau sauvage (TM), Ceraphyl 60 (TM), cremophor Rh 40 (TM), BHA, alpha-bisabolol, carbopol 980 (TM) gel, aqueous excipient qsp, cremophor RH₄O, ethanol, hyaluronic acid, propylene glycal, panthenol, perfumed aqueous excipient qsp, alcohol, oxyacanthine, copra diethanolamine, sodium lauryl ether sulfae and/or any other cosmetic compositions so for used. Among the 18-MP-containing cosmetics or extermal preparations that can be applied to the skin, eye, ear, nose and oral cavity, are there cream, ointment, lotion, gel, emulsion, body milk, powder, shampoo, rinse, hair conditioner, lipstick, tooth paste, soap, gargle (mouth wash), eye wash, ear wash, face cream, hand-cream, body-cream and others. The concentration of 18-MP in these cosmetics or external preparations should be kept low as described in Example 7. Peptide fragments shown in Table 2 and EPO as well as any peptide growth factors, chemokines, cytokines and fragments thereof appear to have the same effect and efficacy as those of 18-MP.

Further, 18-MP with a potent cytoprotective action is useful for cultivating or raising fish, shellfish, lobster, shrimp, eel, crab, oyster, abalone, scallop, conger, rice, wheat, vegetable, fruit and any other marine and agricultural products. 18-MP, if added at the low concentrations to the water or sea water for raising the above vertebrates and invertebrates, can also protect them against endocrine disruptors, toxins, microoganisms, biohazards or traumatic wound. The peptides mentioned in Table 2 and EPO are likely to have the same way of usage as 18-MP.

Since 18-MP and ginsenoside Rb₁, even though their chemical structures are different, exhibit a potent cytoprotective action through upregulation of Bcl-x_{L} expression in the similar low concentration range, 18-MP appears to have the same biological effects and efficasy as those of ginsenoside Rb1 (the Japanese Patent Application Nos. Hei 10-365560, Hei 11-041517, and PCT/JP99/02550) and vice versa. This speculation is reinforced by the finding that intracerebroventricular infusion of 18-MP ameliorates cortical infarction and place navigation disability in stroke-prone spontaneously hypertensive rats with permanent occlusion of the middle cerebral artery distal to the striate branches, and so dose intracerebroventricular infusion of ginsenoside Rb1 (Igase, K. et al., J. Cereb, Blood Flow Metab., 19, 298-306, 1999; Zhang, B. et al., J. Stroke Cerebrovasc. Dis., 7, 1-9, 1998). Moreover, the other ginsenosides similar in chemical structure to ginsenoside Rb1 are also likely to exhibit the same biological actions as those of 18-MP or ginsenoside Rb1 in the low concentration range. It is also plausible that 18-MP at the low concentrations facilitates cell survival by regulating the functions of cell death-related factors apart from Bcl-x_{L}, such as Bcl-2, Bcl-w, Bax, Bik, Bad, Bak, Fas and caspases.
In stroke-prone spontaneously hypertensive rats (SH-SP rats) with permanent occlusion of the middle cerebral artery (MAC) distal to the striate branches, ginsenoside Rb1 has been shown to ameliorate cerebrocortical infarction and ischemia induced place navigation disability through a potent cytoprotective action and through promoting the regeneration and/or reconstruction of cerebral blood vessels once damaged in the ischemic penumbra (the Japanese Patent Application No. Hei 11-041517). On the basis of the finding that 18-MP exerts almost the same effects as ginsenoside Rb1 on the SH-SP rats with permanent MCA occlusion, 18-MP is likely to not only exhibit a potent cytoprotective action but also facilitate the regeneration and/or reconstrucrtion of blood vessels, as does ginsenoside Rb1. It is contemplated that both 18-MP and ginsenoside Rb1 can prevent, cure or treat the cell death-mediated diseases mentioned above by blocking the intracellular cell death machinery and by stimulating the regeneration and/or reconstruction of all kinds of damaged cells, tissues and organs including endothelial cells, fibroblasts, smooth muscle cells, endothelium, basement membrane, connective tissue, tunica intima, tunica media, tunica adventitia within the wall of blood vessels.

Following examples illustrate the present invention, but are not construed as limiting the present invention.

### Example 1

### Experiment for the suppression of apoptosis-like neuron death by intracerebroventricular infusion of 18-MP

Rat-derived 18-MP in Fig. 1 (LSELIINNATEELLIKGL) was used. Mongolian gerbils (12 weeks old, body weight 70 - 80 g) were loaded with 3-minutes transient forebrain ischemia under inhalation anesthesia. Three days (72 hours) later, 18-MP (20 ng/day) was continuously infused into the left lateral ventricle through an osmotic minipump for 4 days. One week after 3 minutes transient forebrain ischemia, they were perfused transcardially with 0.1M phosphate buffer containing 4% paraformaldehyde under pentobarbital anesthesia, and then the brain was dissected out. The brain was embedded in paraffin, sections 5 µm thick were cut and they were subjected to TUNEL staining as described elsewhere (Wen, T.-C. et al., J. Exp. Med., 188, 635-649, 1998). In control animals with 3 minutes transient forebrain ischemia, an equal amount of vehicle (0.01 M phosphate buffered-saline containing 0.1% bovine serum albumin) was infused.

Results are shown in Fig. 2. In Fig. 2, (A) shows TUNEL-positive neurons in the hippocampal CA1 region of a control ischemic animal. (B) shows TUNEL-positive neurons in the hippocampal CA1 region of an ischemic animal infused with 18-MP (20 ng/day). As shown in Fig. 2 (A), in the hippocampal CA1 region of the gerbil with 3-minutes transient forebrain ischemia, many TUNEL-positive neurons were noted at 1 week after the ischemic insult, and the neurons were found to be in the course of apoptosis-like neuron death. When continuous 18-MP infusion into the left lateral ventricle was started at 3 days (72 hours) after 3 minutes transient forebrain ischemia, TUNEL-positive neurons were significantly decreased in number as compared with the control ischemic animals [Fig. 2 (B) and Fig. 2 (C)]. Statistical analysis was performed by Mann-Whitney U-test.

### Example 2

### Effect of 18-MP on long term neuronal degeneration lasting 28 days after brain ischemia

We have further investigated what would happen to the hippocampal CA1 field without 18-MP infusion from 1 week to 1 year after 3-minutes transient forebrain ischemia. Mongolia gerbils were loaded with 3-minutes forebrain ischemia under inhalation anesthesia according to the method of Sakanaka et al. (Sakanaka, M. et al., Proc. Natl. Acad. Sci. USA, 95, 4635-4640, 1998). Step-down passive avoidance learning experiments were performed at 1 week, 1 month, 3 months, 6 months and 12 months after 3-minutes transient forebrain ischemia. Thereafter the individual animals were perfused transcardially with 0.1M phosphate buffer containing 4% paraformaldehyde and 2.5% glutaraldehyde under pentobarbital anesthesia. The brains were dissected out and embedded in paraffin, and then paraffin sections 5 µm thick were cut. All neurons with intact morphological along 1mm linear length of the hippocampal CA1 region were counted in each animal according to the method of Wen et al. (Wen, T.-C. et al., J. Exp. Med., 188, 635-649, 1998). Outlines of the step-down type passive avoidance learning experiment are explained as follows (In details, refer to Wen, T.-C. et al., J. Exp. Med., 188, 635-649, 1998).

One week, one month, 3 months, 6 months and 12 months after 3 minutes transient forebrain ischemia, individual Mongolian gerbils were placed on the safe platform of the passive avoidance learning experiment equipment, but if the gerbils stepped down onto the grid floor, they received foot shock and returned to the safe platform. As a result of 5-minute training, most of the Mongolian gerbils stayed on the safeplatform. 24 hours later, each gerbil was placed on the safe platform while the shock generator was turned off, and the response latency, i. e., the time until it stepped down onto the grid floor, was measured as an index for learning ability.

Fig. 3 shows the results of histopathological analysis of the hippocampal CA1 region. Fig. 3A and Fig. 3a show the hippocampal CA1 field of a sham-operated gerbil at low magnification (A) and at high magnification(a). Fig. 3B and Fig. 3b show the hippocampal CA1 field of a gerbil at 1week after 3 minutes transient forebrain ischemia at low magnification (B) and at high magnification (b). At this time nearly one half of hippocampal CA1 pyramidal neurons degenerated and disappeared. Fig. 3C and Fig. 3c show the hippocampal CA1 field of a gerbil at one month after 3-minutes transient forebrain ischemia at low magnification (C) and at high magnification (c). At this period the hippocampal CA1 pyramidal neurons decreased further in number. Fig. 3D, Fig. 3d, Fig. 3E, Fig. 3e, Fig. 3F and Fig. 3f, show the hippocampal CA1 field of gerbils at 3 months, 6 months and 12 months (one year) after 3 minutes transient forebrain ischemia at low magnification (D, E, F) and at high magnification (d, e, f), respectivery. At these periods, large differences could not be observed.

Table 1 shows quantified data of the results in Fig. 3 and the response latency of the passive avoidance learning experiment. Results indicate that the neuronal density in the hippocampus CA1 region reduced to approximately one half at one week after 3-minutes transient forebrain ischemia, and they further decrease in number until one month after ischemia. Thereafter, no significant changes in the neuronal density of the hippocampal CA1 region were noted. Table 1 also shows the response latency in the step-down passive avoidance learning tests in the sham-operated group, and at 7 days (one week), one month, 3 months, 6 months and one year (12 months) after 3-minutes transient forebrain ischemia. The learning ability was significantly lowered at 7 days (1 week) after 3-minutes transient forebrain ischemia as compared with the learning ability of the sham-operated group. One month after 3-minutes forebrain ischemia, the response latency was further significantly shortened. No significant changes in the response latency were noted between 3 months and 6 months after 3-minutes transient forebrain ischemia. One year (12 months) later, in spite of showing no changes in the hippocampal CA1 pyramidal neuronal density as compared with that of 6 months, only the response latency was significantly reduced. Statistical analysis was performed by Student's t test with Bonferroni correction for multiple comparison.

Next, 18-MP was continuously infused into the lateral ventricle of Mongolian gerbils in a dose of 20 ng/day from 3 days to 7 days after 3-minutes transient forebrain ischemia, and the effects of 18-MP were studied. Three minute forebrain ischemia was loaded to Mongolian gerbils under inhalation anesthesia, and 3 days (72 hours) later, 18-MP (20 ng/day) was continuously infused into the left lateral ventricle for 4 days through an osmotic minipump. The step-down type passive avoidance learning experiments were conducted as described in our previous studies (Wen, T.-C. et al., J. Exp. Med., 188, 635-649, 1998; and Sakanaka, M. et al., Proc. Natl. Acad. Sci. USA, 95, 4635-4640, 1998). Mongolian gerbils were perfused transcardially with 4% paraformaldehyde and 2.5% glutaraldehyde in 0.1M phosphate buffer under pentobarbital anesthesia, and the brains were dissected out. The brains were embedded in paraffin, and paraffin sections 5 µm thick were cut. All neurons with intact morphological appearance along 1mm linear length of the hippocampal CA1 region were counted according to the method of Wen et al. and Sakanaka et al. (Wen, T.-C. et al., J. Exp. Med., 188, 635-649, 1998; and Sakanaka, M. et al., Proc. Natl. Acad. Sci. USA, 95, 4635-4640, 1998). The equal amount of vehicle (0.01 M phosphate buffered-saline containing 0.1% bovine serum albumin) was infused in the control ischemic animals and in the sham-operated animals.

In Fig. 4A, columns with slanting lines show the neuronal density of the hippocampal CA1 region of 18-MP-infused ischemic animals and non-infused ischemic animals. In Fig. 4B, columns with slanting lines show response latency of the passive avoidance learning experiments in 18-MP-infused ischemic animals and non-infused ischemic animals. Significant increases in the neuron density and the response latency were observed in animals infused with 18-MP from 3 days to 7 days after 3-minutes transient forebrain ischemia, as compared with non-infused ischemic animals. Fig. 4C and Fig. 4D show the hippocampal CA1 regions of a non-infused ischemic animal and an ischemic animal with 4 day 18-MP infusion, respectively. 18-MP infusion starting at 3 days after 3-minutes transient forebrain ischmia apparently rescued a large number of hippocampal CA1 pyramidal neurons. In Fig. 4A and Fig. 4B, open columns show the experimental results of animals with sham operation. As explained above, only 4 day intracerebroventricular infusion of 18-MP starting at 3 days (72 hours) after 3-minutes forebrain ischemia rescued a large number of hippocampal CA1 neurons at one week (7 days) after ischemia. In addition, as shown in Fig. 2, TUNEL-positive neurons at this point were significantly less numerous in the 18-MP administered than in non-administered group. It is plausible that 18-MP infusion prevents degeneration of the hippocampal CA1 neurons, which occurs after one week (7 days) of 3-minutes ischemic insult. Statistical analysis was performed according to Mann-Whitney U test.

Next, 18-MP was continuously infused into the cerebral ventricle from 3 days to 31 days after 3-minutes transient forebrain ischemia, and the effect of the 28-day 18-MP administration was investigated. Namely, Mongolian gerbils were loaded with 3-minutes transient forebrain ischemia under inhalation anesthesia. At 3 days (72 hours) after ischemia, 18-MP (20 ng/day) was continuously infused into the left lateral ventricle through an osmotic minipump for 28 days. Thereafter conventional step-down passive avoidance learning experiments were performed (Wen, T.-C. et al., J. Exp. Med., 188, 635-649m 1998; and Sakanaka, M. et al., Proc. Natl. Acad. Sci. USA, 95, 4635-4640, 1998), and Mongolian gerbil were perfused transcardially with 0.1M phosphate buffer containing 4% paraformaldehyde under pentobarbital anesthesia, and the brain was disseced out. The brain was embedded in paraffin, and paraffin sections 5 µm thickn were cut. All neurons with intact morphological appearance along 1mm linear length of the hippocampal CA1 region, were counted according to the method of Wen et al. and Sakanaka et al. (Wen, T.-C. et al., J. Exp. Med., 188, 635-649, 1998; Sakanaka, M. et al., Proc. Natl. Acad. Sci. USA, 95, 4635-4640, 1998). For the control animals, the equal amount of vehicle (0.01 M phosphate buffered-saline containing 0.1 % bovine serum albumin) was administered.

Results are shown in Fig. 4A, B, E and F. As shown in the closed columns of Fig. 4A and B, the 28 day 18-MP infusion in a dose of 20 ng/day significantly prolonged response latency in the passive avoidance learning exp eriment and rescued a significant number of hippocampal CA1 neurons as compared with the vehicle-infused ischemic group (i.e. 0 ng/day of 18-MP-infused group). Fig. 4E shows a photomicrograph of the hippocampal CA1 region of an ischemic animal infused with vehicle from 3 days (72 hours) to 31 days after 3-minutes forebrain ischemia and Fig. 4F shows a photomicrograph of the hippocampal CA1 region of an ischemic animal infused with 18-MP (20 ng/day) in the same schedule. Therefore, a significant number of hippocampal CA1 pyramidal neurons is rescued by the 28-days administration of 18-MP (20 ng/day) that starts at 3 days after ischemia. Administration of 18-MP in a dose of 4 ng/day showed no significant effect. NS means no significant difference. Statistical analysis was performed according to Mann-Whitney U test.

### Example 3

### Experiment on the suppressive action of 18-MP on neuron death in vitro

Neurons from the cerebral cortices of 17-day-old rat embryos were incubated in a serum-free medium for 4 or 5 days, and the medium was replaced with the fresh medium with or without 18-MP, and the neurons were further cultured for 24 hours. Thereafter, A nitric oxide donor, sodium nitroprusside (SNP) at a concentration of 100 µM, was added to the medium for 10 minutes. Then the neurons were incubated in the medium containing 18-MP and survival rate of the neurons was measured with the use of a redox indicator, alamer blue, according to the method of Sakanaka, Tanaka and Toku et al. (Toku, K. et al., J. Neurosci. Res., 53, 415-425, 1998).

Results are shown in Fig. 5. The closed columns on the left side of Fig. 5 indicate the survival rate of neurons with or without addition of 18-MP without SNP treatment. The closed columns on the right side of Fig. 5 indicate the survival rate of neurons with addition of 18-MP before and after SNP treatment. The columns with slanting lines on the right side of Fig. 5 indicate the survival rate of neurons with addition of 18-MP after SNP treatment. As shown on the left side of Fig. 5, without SNP treatment, 18-MP did not significantly affect the metabolic activity of cultured neurons. When neurons were treated with only SNP without addition of 18-MP, apoptosis-like neuron death or apoptosis of neurons occurred as shown in the column with wave lines on the right side of Fig. 5. 18-MP significantly suppressed, even in the concentration range of 1 - 100 fg/ml, apoptosis-like neuron death (apoptosis of neurons) not only in the case of 18-MP addition before and after SNP treatment but also in the case of 18-MP addition after SNP treatment.

* p<0.05, ** p<0.01, *** p<0.001 indicate significant differences. Statistical analysis was performed according to ANOVA+Fisher PLSD.

### Example 4

### Experiment on the effect of 18-MP on Bcl-x_{L} protein expression in ncurons

In order to investigate whether low concentrations of 18-MP enhance the expression of Bcl-x_{L} protein in nerve cells or neurons, the western blotting technique was applied with the use of anti-Bcl-x_{L} protein antibody. Neurons from the cerebral cortices of 17-day-ofd rat embryos were cultured for 48 hours with or without the presence of the low concentrations of 18-MP. The neurons were lysed in sample buffer for electrophoresis and electrophoresed. Then the electrophoretic proteins were transferred to nitrocellulose membrane and subjected to western blotting. Results are shown in Fig. 6.

Further, bands reacted with the anti-Bcl-x_{L} protein antibody were quantified by image analysis. Results are shown in Fig. 7. Details of the experimental method are described by the inventors (Sakanaka, Tanaka and Satoh) and Wen et al. (Wen, T.-C. et al., J. Exp. Med., 188, 635-649, 1998).

As shown in Fig. 6 and Fig. 7, 18-MP significantly increased the expression of Bcl-x_{L} protein in neurons within the concentration range of 1 - 100 fg/ml. In the same concentration range, 18-MP suppresses apoptosis-like neuron death or apoptosis of neurons as shown in Fig. 5. Statistical analysis was performed according to ANOVA+Fisher's PLSD.

### Example 5

### Analysis of stimulation by erythropoietin (EPO) of Bcl-x_{L} expression

Experimental techniques are the same as those described in the study of the inventors (Sakanaka, Tanaka and Satoh) and Wen et al. (Wen, T.-C. et al., J. Exp. Med., 188, 635-649, 1998). Neurons from cerebral cortices of 17-day-old rat embryos were cultured in a medium containing 10 % fetal calf serum. On the 2nd day of culture, the medium was replaced with a serum-free medium, and on the 4th day of culture, EPO (0, 10, 10³, or 10⁶ fg/ml) was added to the medium, then the neurons were cultured for 24 hours. Thereafter total RNA was extracted from the cultured neurons. cDNA was prepared from DNAase-treated total RNA (3 µg) by using oligo dT primer and reverse transcriptase. PCR was conducted with the use of Taq polymerase in the following condition, i.e. 20 cycles (for p-actin) or 25 cycles (for Bcl-x_{L} ) at (1) 94°C, 2 min.; (2) a cycle with 94°C, 1.5 min.; 55°C, 1.5 min.; 72°C, 2 min.

The PCR products were electrophoresed on 3 % agarose gel and visualized with ethidium bromide. Expression of β-actin mRNA was used as an internal standard. The results are shown in Fig. 8. As shown in Fig. 8, the expression of Bcl-x_{L} mRNA was enhanced in neurons treatmed with EPO (10³ and 10⁶ fg/ml) as compared with neurons without EPO treatment. Especially, in neurons treated with 10³ fg/ml of EPO, the expression of Bcl-x_{L} mRNA was markedly increased.

We have further investigated whether EPO could enhance the expression of Bcl-x_{L} protein in primary cultured neurons. Neurons from the cerebral cortices of 17-days-old rat embryos were cultured for 48 hours with EPO (10, 10³, or 10⁶ fg/ml) or without EPO. Cells were lysed with sample buffer for electrophoresis and subjected to electrophoresis. Electrophoresed proteins were transferred to nitrocellulose membrane and western blotting with the anti- Bcl-x_{L} antibody was perforated. The results are shown in Fig. 9. As shown in Fig. 9, the expression of Bcl-x_{L} protein was increased in neurons treated with EPO at concentrations of 10³ and 106 fg/ml as compared with neurons without EPO treatment, and especially at the concentration of 10³ fg/ml, EPO stimulated Bcl-x_{L} protein expression the most effectively. The effect of EPO at a concentration of 10 fg/ml on Bcl-x_{L} protein expression was not clear.

### Example 6

### Experiment on the protective action of 18-MP on cardiac muscle cells

We have investigated whether or not 18-MP protected cardiac muscle cells and enhanced Bcl-x_{L} protein expression in the said cells.

Hearts were dissected out from 17-days-old rat embryos and the atrium was removed. The tissues were cut into small pieces with scissors, and incubated with trypsin-EDTA solution at room temperature for one hour to disperse cells. In order to remove contaminated fibroblasts, the cell suspension was put into conventional dishes for cell culture and incubated for 20 minutes. Non-adhered cells were seeded on a 24-well plate for primary culture (Falcon Inc.) and cultured in DMEM (Dulbecco's modified Eagle's medium) containing 10% FCS (fetal calf serum).

18-MP was added to the cultured cardiac muscle cells on the 24 well plate at 0.01 fg/ml - 1 ng/ml. 24 hours later, homogenates were prepared, electrophoresed and subjected to western blotting by using the anti-Bcl-x_{L} antibody. Troponin T, which was only expressed in the striated muscle, was used as an internal standard. Bands reacted with the anti-Bcl-x_{L} antibody were quantified with an image analyger. In addition, cultured cardiac muscle cells (myocytes) in the 24-well plate were maintained in a culture medium devoid of glucose, to which 18-MP was added at the concentrations of 0.01 fg/ml - 1 ng/ml. After cultured for 4 - 5 days, the cardiac myocytes in each well were lysed and subjected to western blotting with an antibody against striated muscle-specific α-actinin to estimate the survival rates of cardiac myocytes that were treated with different concentrations of 18-MP. The results are shown in Fig. 10, Fig. 11 and Fig. 12.

As shown in Fig. 10 and Fig. 11, 18-MP, in the concentration range between 1 fg/ml and 100 fg/ml, significantly enhanced the expression of Bcl-x_{L} protein in cardiac muscle cells (myocytes). Since there was no change in the amount of troponin T detected in each well of the 24-well plate, 18-MP appeared to stimulate the expression of Bcl-x_{L} protein without affecting the number or survival rate of cardiac myocytes during the 24 hour cultivation.

As shown in Fig. 12, 4 - 5 days treatment of cardiac muscle cells in a glucose-free mediun with 18-MP at concentrations of 10⁻² - 10⁴ fg/ml apparently suppressed a decline in the amount of striated muscle-specific α-actinin as detected by western blotting. When cultured only with the glucose-free medium devoid of 18-MP, cardiac myocytes almost disappeared within 4-5 days. On the contrary large numbers of cardiac myocytes were observed in the wells treated with 18-MP at the concentrations of 0.01 fg/ml, 1 fg/ml, 100 fg/ml and 10 pg/ml. Further in the presence of a higher concentration of 18-MP (10⁶ fg/ml) the survival of cardiac muscle cells was not facilitated.

### Example 7

### Prevention, therapy or treatment of bedsore or chronic pressure sore by 18-MP

Bed sore of bedridden patients and aged people induces deterioration of the general condition and bedsore is a skin disease markedly damaging QOL (quality of life). At the early stage of bedsore, flare of skin lesion is observed, but there are almost no drugs for external use showing effectiveness and efficacy at this stage by applying for the local and peripheral lesions. This is a serious problem in the dermatological field.

External preparation for skin (cream, gel or ointment) was prepared by mixing 18-MP in water-soluble base or fat-soluble base with or without addition of glucose, vitamine E, steroids, vitamine D, vitamine D derivatives, antiviral agents, anti-allergic agents, immunosuppressants, antibiotics and/or any other dermatologic compositions so far used. The preparation is applied regularly on bedsore lesion and its peripheral field until obtaining cure or reduction of bedsore lesion, or until showing no deterioration of bedsore lesion. The amount of mixing 18-MP in the external preparafion for skin is controlled so that the extracellular levels of 18-MP in the local lesion is at 1 ng/ml (approximately 0.5 nM) or less, preferably 10 pg/ml (approximately 5 pM) or less, more preferably 100 fg/ml (approximately 50 fM) or less. Furthermore, continuous or single intravenous infusion of 18-MP may be combined with the external application of 18-MP to the skin for the treatment, therapy or prevention of bedsore. The concentration of 18-MP in the external preparation for skin or in any cosmetics (cream, ointment, lotion, lipstick, tooth paste, gel, emulsion, body milk, rinse, gargle, mouth wash, shampoo, powder, soap, hair conditioner and so on) is 0.1 % or less, preferably 0.001 % or less, more preferably between 10⁻⁵ % and 10⁻¹⁵ % by weight, based on the total weight of the composition. The uppermost concentration of 18-MP in the external preparation for skin or in cosmetics is 1 %.

The above method for the treatment, therapy or prevention of bedsore can be applied to any types of skin diseases including skin ulcer, traumatic wounds, nontraumatic wounds and burns. Preferably, 18-MP is quite useful for promoting wound healing and for protecting the skin from ultraviolet ray, radiation or sunlight.

### Example 8

### Prevention, therapy or treatment of corneal injury

It is well known to develop corneal injury after applying contact lens or orthotic operation of myopia by excimer laser, however there are almost no eye drops or ointments for protecting corneal tissues against such injuries.

Eye drops or ointments are prepared by mixing 18-MP with various solutions or bases for eye drops and are applied consecutively to the patients with corneal injury for necessary period and times every day until improvement or cure of the comeal lesion is observed. In the application of eye drops, the amount of 18-MP mixed with the basal solution or substance for eye drops should be controlled so that the extracellular concentrations of 18-MP in the corneal lesion are kept at 1 ng/ml (approximately 0.5 nM) or less, preferably 10 pg/ml (approximately 5 pM) or less, and more preferably 100 fg/ml (approximately 50 fM) or less.

### Example 9

### Protection of cornea for keratoplasty by using 18-MP

Keratoplasty is frequently performed in the ophthalmological field as treatment with the highest success rate in the transplantation medical field. However, cells of the cornea for keratoplasty are gradually enter apoptosis-like death during the term from collection of the cornea of a donor to the operation. This is a largest critical path for restricting time from collection of the cornea to keratoplasty.

After collection of the cornea for keratoplasty, 18-MP is added to the conventional preservatives for the cornea at 1 ng/ml (approximately 0.5 nM) or less, preferably 10 pg/ml (approximately 5 pM) or less, and more preferably 100 fg/ml (approximately 50 fM) or less.

## Claims

1. Use of a prosaposin-related peptide or derivative thereof comprising an amino acid sequence
J-(X)ₘ-N-N-(X)ₙ-(O)ₚ-(X)_{q}-(U)ᵣ
wherein
J is Leu or Ile;
X is any amino acid residue;
O is Asp, Lys, Glu or Arg;
U is Ala, Leu, Ile or Val;
m is 1, 2 or 3
n is 1 to 7;
p is one, two or more;
q is 5 to 9 and
r is one, two or more,
in the production of a medicament for use in preventing or delaying cell death or in promoting the expression of the cell death supporting gene product Bcl-X_{L}.

2. Use of two or more prosaposin-related peptides or derivatives thereof according to claim 1.

3. A process of preventing death of cells *in vitro* or *ex vivo* comprising administering to the cells two or more prosaposin-related peptides or derivatives thereof as defined in claim 1.

4. A pharmaceutical composition comprising two or more prosaposin related peptides or derivatives thereof as defined in claim 1.

5. Use of a prosaposin-related peptide or derivative thereof comprising an amino acid sequence 
SEQUENCE LISTING

<110> SAKANAKA, Masahiro
      TANAKA, Junya
      SATO, Kohji

<120> CYTOPROTECTIVE AGENTS COMPRISING PROSAPOSIN-RELATED
      PEPTIDES

<130> N79825 TAC AB

<140> 00305504.3
<141> 2000-06-30

<150> JP 11-185155/1999
<151> 1999-06-30

<160> 20

<170> PatentIn Ver. 2.1

<210> 1
<211> 8
<212> PRT
<213> Artificial Sequence

<220>
<221> VARIANT
<222> (1)
<223> Xaa is Leu or Ile

<220>
<221> VARIANT
<222> (2)
<223> Xaa is  1, 2 or 3 amino acid residues, each being
      any possible amino acid residue

<220>
<221> VARIANT
<222> (5)
<223> Xaa is 1 to 7 amino acid residues, each being any
      possible amino acid residue

<220>
<221> VARIANT
 wherein Xaa is any amino acid residue
in the production of a medicament for use in preventing or delaying cell death or in promoting the expression of the cell death supporting gene product Bcl-X_{L}.

6. Use of two or more prosaposin-related peptides or derivatives thereof according to claim 5.

7. A process of preventing death of cells *in vitro* or *ex vivo* comprising administering to the cells two or more prosaposin-related peptides or derivatives thereof as defined in claim 5.

8. A pharmaceutical composition comprising two or more prosaposin-related peptides or derivatives thereof as defined in claim 5.

9. Use of a prosaposin-related peptide or derivative thereof comprising an amino acid sequence in the production of a medicament for use in preventing or delaying cell death or in promoting the expression of the cell death supporting gene product Bcl-X_{L}.

10. Use of two or more prosaposin-related peptides or derivatives thereof according to claim 9.

11. A process of preventing death of cells *in vitro* or *ex vivo* comprising administering to the cells two or more prosaposin-related peptides or derivatives thereof as defined in claim 9.

12. A pharmaceutical composition comprising two or more prosaposin-related peptides or derivatives thereof as defined in claim 9.

13. Use according to any one of claims 1, 2, 5, 6, 9 and 10 or a process according to any one of claims 3, 7 and 11 wherein sufficient prosaposin-related peptides or derivatives thereof are administered to achieve an extracellular-fluid concentration of prosaposin-related peptide or derivative thereof of 1 ng/ml (approximately 0.5 nM) or less.

14. Use according to any one of claims 1, 2, 5, 6, 9, 10 and 13 or a process according to any one of claims 3, 7, 11 and 13 wherein the cells are brain cells, neurons or cardiac muscle cells.

15. Use according to any one of claims 1, 2, 5, 6, 9, 10, 13 and 14 or a process according to any one of claims 3, 7, 11, 13 and 14 wherein erythropoietin or a salt thereof is administered separately, simultaneously or sequentially, or co-administered with the prosaposin-related peptides or derivatives thereof.

16. Use or a process according to claim 15 wherein sufficient erythropoietin or a salt thereof is administered to achieve an extracellular fluid concentration or erythropoietin or salt thereof of 1 ng/ml (approximately 10⁶ fg/ml) or less.

17. A pharmaceutical composition comprising at least one prosaposin-related peptide or derivative thereof as defined in any one of claims 1, 5 and 9 together with erythropoietin and a pharmaceutically acceptable carrier.
